# FASCICULE DE BREVET EUROPEEN

(11) **EP 2 307 116 B1**
(45) Date de publication et mention de la délivrance du brevet: **21.03.2012**
(21) Numéro de dépôt: 09754069.4
(22) Date de dépôt: 28.05.2009
(51) Int. Cl.: B01D 11/04, C07C 39/17

(54) **PROCEDE D'EXTRACTION SELECTIVE DE PROTEINES MEMBRANAIRES AVEC LES CALIXARENES**
VERFAHREN ZUR SELEKTIVEN EXTRAKTION VON MEMBRANPROTEINEN UNTER VERWENDUNG VON CALIXARENEN
METHOD FOR SELECTIVELY EXTRACTING MEMBRANE PROTEINS USING CALIXARENES

(30) Priorité: 28.05.2008 FR 0802894
(43) Date de publication de la demande: 13.04.2011
(73) Titulaire: Centre National de la Recherche Scientifique - CNRS, 75794 Paris Cedex 6 (FR); Universite Claude Bernard de Lyon 1, 69622 Villeurbanne Cedex (FR)
(72) Inventeur: COLEMAN, Anthony, William, F-69300 Caluire (FR); MBEMBA, Cyrille, F-69001 Lyon (FR); FALSON, Pierre, F-07100 Annonay (FR); MATAR, Rima, F-69001 Lyon (FR); HUCHÉ, Frédéric, F-91590 La Ferte Alais (FR)
(74) Mandataire: Novagraaf Technologies
(86) Numéro de dépôt international: PCT/FR2009/000626
(87) Numéro de publication internationale: WO 2009/144419

(56) Documents cités:
- EP-A- 0 954 965
- WO-A-89/08092
- OSHIMA, HIGUCHI,OHTO, INOUE, GOTO: "Selective Extraction and Recovery of Cytochrome c by Liquid-Liquid Extraction Using a Calix[6]arene Carboxylic Acid Derivative" LANGMUIR, vol. 21, 30 juin 2005 (2005-06-30), pages 7280-7284, XP002502667
- MARTINEZ-ARAGON, GOETHEER, DE HAAN: "Host-guest extraction of immunoglobulin G using calix[6]arenas" SEPARATION AND PURIFICATION TECHNOLOGY, 26 mars 2008 (2008-03-26), XP002502668
- GOTO M. ET AL: "Recent advances in Protein Extraction and Chrial Separation of Biomolecules" TSINGHUA SCIENCE AND TECHNOLOGY, vol. 11, no. 2, avril 2006 (2006-04), pages 194-201, XP005350941 TSINGHUA UNIVERSITY PRESS, BEIJING, CN ISSN: 1007-0214

## Description

### Domaine technique

La présente invention se rapporte à un procédé d'extraction sélective de protéines membranaires avec au moins un calixarène.

L'utilisation des calixarènes dans le procédé selon l'invention permet de solubiliser sélectivement les protéines membranaires tout en préservant leur structure tridimensionnelle qui est essentielle à leur activité enzymatique.

Dans la description ci-dessous, les références entre crochets [ ] renvoient à la liste des références présentée à la fin du texte.

### État de la technique

L'extraction des protéines membranaires est une étape prérequise pour leur étude en solution, notamment leur caractérisation structurale par radiocristallographie, qui implique de les maintenir en solution dans un état topologique identique à celui qu'elles adoptent dans les membranes.

En général, cette étape est réalisée à l'aide de détergents ou tensioactifs qui, d'une part, permettent d'extraire ces protéines de leur environnement membranaire, déstructurant la bicouche membranaire en entrant en compétition avec les chaînes aliphatiques des lipides qui la constituent et, d'autre part, maintiennent la protéine membranaire en solution du fait de leur composante hydrophile. Cependant, ces détergents ne présentent pas les mêmes caractéristiques physico-chimiques que les lipides et donc tendent à déstabiliser la structure des domaines membranaires, ce qui est une source d'hétérogénéité structurale.

Bien qu'étant les plus efficaces pour solubiliser les protéines membranaires, les détergents ou tensioactifs anioniques, comme par exemple le sodium dodécyle sulfate (SDS) ou le désoxycholate de sodium, sont généralement déstructurants. Par ailleurs, l'efficacité de la phase de déstructuration de la bicouche lipidique est fonction de la longueur de la chaîne aliphatique du détergent ou tensioactif, un paramètre qui varie en fonction de l'origine des membranes et des protéines qui y sont insérées.

Par ailleurs, diverses études concernant les intéractions de divers calixarènes avec des protéines sont connues mais elles ne permettent pas d'extraire des protéines membranaires de leur environnement membranaire, et notamment sans dénaturation de celles-ci.

Le document Oshima f. et al. (LANGMUIR, vol.21, pages 7280-782, 2005) décrit l'utilisation d'un calix[6]arène spécifique pour l'extraction sélective du cytochrome c.

WO 89/08092 enseigne que certain calix[4-8]arènes sont aptes à extraire des hydrocarbures.

Dans le cadre de la présente invention, les termes « tensioactif », « surfactant » et « détergent » sont indifféremment utilisés pour désigner des molécules ayant des propriétés particulières dues à leur structure amphiphiles. Une molécule est dite amphiphile lorsqu'elle possède à la fois un groupe polaire et hydrophile et un groupe apolaire et hydrophobe.

Le terme « extraction sélective » dans la présente invention signifie un procédé permettant d'extraire les protéines membranaires, sélectivement les unes par rapport aux autres, d'un environnement membranaire par solubilisation des membranes lipidiques dans lesquelles ces protéines sont insérées, qu'elles soient naturelles ou artificielles, et la mise en solution desdites protéines.

On entend par « solubilisation » ou « mise en solution » des protéines membranaires, au sens de la présente invention, le passage de la protéine membranaire de l'environnement membranaire, c'est-à-dire d'un milieu essentiellement lipidique, à l'environnement aqueux. Dans l'environnement aqueux, les protéines « solubilisées » peuvent être en suspension, en dispersion ou sous forme de molécules non libres dans la solution aqueuse. Lesdites protéines peuvent éventuellement être associées ou liées à des surfactants ou détergents.

A ce jour, il existe très peu de détergents polyanioniques non dénaturants pour les protéines membranaires permettant leur extraction sélective.

II existe donc un réel besoin d'un procédé d'extraction palliant les défauts, limitations, inconvénients et obstacles de l'art antérieur, en particulier d'un procédé mettant en oeuvre des molécules anioniques ayant des propriétés détergentes permettant d'extraire sélectivement les protéines membranaires sans les dénaturer, c'est-à-dire sans les destructurer ou modifier leur conformation tridimensionnnelle normale qui leur permet de remplir leur fonction.

### Description de l'invention

La présente invention a précisément pour but de répondre à ce besoin en fournissant un procédé d'extraction sélective de protéines membranaires comprenant une étape qui consiste à mettre en contact une solution aqueuse de la protéine membranaire à extraire avec au moins un calixarène de formule (I) : 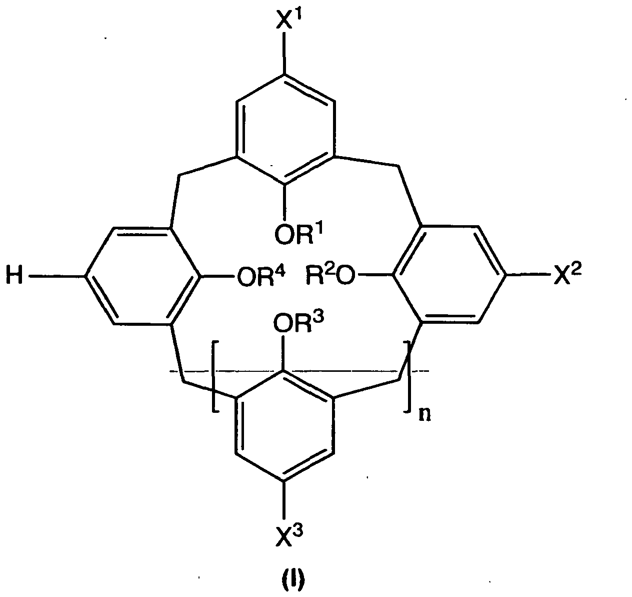 dans laquelle :
■ n est un nombre entier égal à 1, 3, 5 ou 6 ;
■ R¹, R², R³, R⁴ représentent, indépendamment l'un de l'autre, un atome d'hydrogène ; un groupe (C₁₋₁₂) alkyle (par exemple (C₃₋₁₂) alkyle, par exemple (C₉₋₁₂) alkyle) linéaire ou ramifié éventuellement substitué par un ou plusieurs hétéroatomes choisis dans le groupe d'atomes O, S, N, P ; un groupe (C₁₋₁₂) alkyle (par exemple (C₃₋₁₂) alkyle, par exemple (C₉₋₁₂) alkyle), linéaire ou ramifié éventuellement substitué par un groupe -COOR où R est un groupe (C₁₋₄) alkyle linéaire ou ramifié ; ou un groupe aryle comprenant de 6 à 20 atomes de carbone ;
■ X¹, X², et X³ représentent, indépendamment l'un de l'autre, un groupe - (CH₂)ₘ-COOR' dans lequel
   - m est un nombre entier allant de 0 à 10,
   - R' représente un atome d'hydrogène ou un groupe (C₁₋₄) alkyle linéaire ou ramifié ;
ou l'un de ses sels pharmaceutiquement acceptables.

Selon un mode de réalisation particulier de l'invention, le procédé d'extraction sélective de la protéine membranaire peut être réalisée avec au moins un calixarène de formule (I) dans laquelle :
■ n est un nombre entier égal à 1 ;
■ R¹, R², R³, R⁴ représentent, indépendamment l'un de l'autre, un atome d'hydrogène ; un groupe (C₁₋₁₂) alkyle linéaire ou ramifié ;
■ X¹, X² et X³ représentent, indépendamment l'un de l'autre, un groupe - (CH₂)ₘ-COOR' dans lequel
   - m est un nombre entier allant de 0 à 10,
   - R' représente un atome d'hydrogène ;
ou l'un des sels pharmaceutiquement acceptables.

On entend par « alkyle » au sens de la présente invention, un radical carboné linéaire, ramifié ou cyclique, saturé ou insaturé, éventuellement substitué, comprenant 1 à 12 atomes de carbone. À titre indicatif, on peut citer les radicaux méthyle, éthyle, propyle, butyle, isobutyle, pentyle, hexyle, octyle, nonyle, décyle, undécyle, dodécanyle et leurs isomères ramifiés. Le groupe alkyle peut être éventuellement substitué par un ou plusieurs hétéroatomes choisis dans le groupe d'atomes O, S, N, P.

Le terme « aryle » désigne de manière générale un substituant aromatique cyclique comportant de 6 à 20 atomes de carbone. Dans le cadre de l'invention le groupe aryle peut être mono- ou poly- cyclique éventuellement substitué. À ce titre on peut citer le benzyle, le phényle.

Dans le cadre de la présente invention, le terme « sels pharmaceutiquement acceptables » comprend les sels préparés avec des acides ou bases, non toxiques, en fonction des substituants présents sur les composés. Lorsque les composés de l'invention comportent des fonctions acides, les sels correspondants peuvent être obtenus par addition d'une base organique ou inorganique sur le composé sous forme neutralisée en présence éventuellement dans un solvant de préférence inerte. Des exemples de sel d'addition d'une base peuvent être les sels de sodium, potassium, calcium, ammonium, amino (organique), ou magnésium. Lorsque les composés de l'invention comportent des fonctions basiques, les sels correspondants peuvent être obtenus par addition d'un acide organique ou inorganique éventuellement dans un solvant de préférence inerte. Des exemples de sels d'additions d'acide inorganique peuvent être les sels hydrochlorique, hydrobromique, nitrique, carbonique, monohydrogéncarbonique, phosphorique, monohydrogénphosphorique, dihydrogénophosphorique, sulfurique, monohydrogénosulfurique hydroiodique. Des exemples de sels d'additions d'acide organique peuvent être les sels acétique, propionique, isobutyrique, maléique, malonique, benzoïque, succinique, subérique, fumarique, lactique, mandélique, phthalique, benzénesulfonique, p-tolylsulfonique, citrique, tartarique, méthanesulfonique.

On entend par « protéine membranaire » au sens de la présente invention une protéine associée aux membranes biologiques, c'est-à-dire soit ancrées, soit intégrales, et non libre de diffusion dans les milieux aqueux et instable dans ces milieux. Parmi les protéines membranaires, on peut citer par exemple les protéines de membranes plasmiques et les protéines de membranes intracellulaires (comme par exemple les protéines de membranes mitochondriales, nucléaires, lysosomales, etc.).

Les protéines membranaires sont souvent classées en fonction des structures qui leur permettent d'interagir avec les membranes et de la manière dont ces structures s'agencent. Les protéines membranaires peuvent être des protéines polytopiques ou des protéines monotopiques, par exemple des protéines polytopiques.

On entend par « protéines polytopiques » des protéines qui traversent une ou plusieurs fois la membrane.

On entend par « protéines monotopiques » des protéines qui interagissent avec un seul coté de la membrane.

Les protéines membranaires peuvent également être classées en fonction de leur difficulté à être extraites des membranes. Elles peuvent être intégrales ou périphériques, par exemple intégrales.

On entend par « protéines intégrales » des protéines monotopiques ou polytopiques qui interagissent fortement avec la membrane, notamment par des interactions hydrophobes. Ces protéines sont aussi appelées « protéines intrinsèques ».

On entend par « protéines périphériques » des protéines monotopiques qui interagissent faiblement avec la membrane, c'est-à-dire soit par des liaisons électrostatiques, soit par l'intermédiaire d'autres protéines membranaires. Ces protéines sont aussi appelées « protéines extrinsèques ».

On entend par « solution aqueuse de la protéine membranaire » au sens de la présente invention, une solution aqueuse comprenant une ou plusieurs protéine(s) membranaire(s). Il peut par exemple s'agir d'une suspension ou d'une dispersion, les protéines pouvant être sous une forme non dissoute ou non diffuse ou pouvant par exemple être combinées à une fraction membranaire biologique.

Les calixarènes de formules (I) sont des molécules anioniques ayant des propriétés détergentes. Ils disposent d'une face hydrophile constituée de plusieurs fonctions carboxyliques et d'une face hydrophobe constituée de groupes alkyles. La multiplicité de charges négatives favorise d'une part l'interaction ionique de la molécule détergente avec la surface hydrophile de la protéine et d'autre part prévient sa pénétration (déstructurante) au coeur (hydrophobe) des protéines.

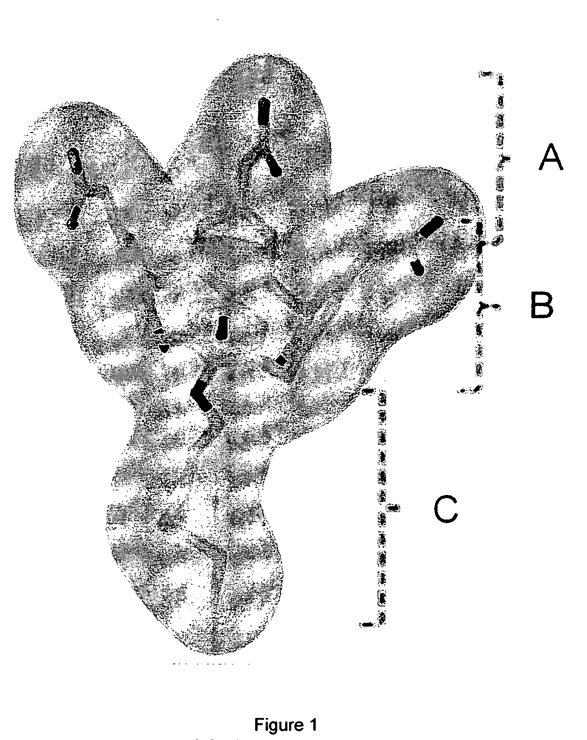

Plus précisément, de par leur formule chimique, les calixarènes de formule (I) ont une géométrie moléculaire sous forme de cône ou de tronc de cône, dans lequel la région évasée est hydrophile tandis que la queue est hydrophobe (figure 1). Cette forme particulière des calixarènes de formule (I) permet la formation de micelles, qui d'une part favorisent le passage des protéines d'un environnement membranaire vers un milieu aqueux, et d'autre part permettent la conservation de leur structure tridimensionnelle et donc de leur activité.

Ainsi, l'utilisation des calixarènes de formule (I) dans le procédé selon l'invention permet d'extraire délectivement les protéines membranaires en les solubilisant sélectivement (c'est-à-dire en permettant leur passage de l'environnement membranaire à l'environnement aqueux) tout en préservant leur structure tridimensionnelle qui est essentielle à leur activité, par exemple enzymatique. Ainsi, après leur reconstitution dans les lipides naturels ou synthétiques, les protéines membranaires retrouvent leur activité.

Par ailleurs, le procédé de l'invention permet l'extraction d'une protéine donnée, sélectivement par rapport à d'autres protéines membranaires, notamment en modulant la longueur de la queue hydrophobe du calixarène. Le procédé de l'invention permet ainsi l'extraction et la séparation de fractions de protéines membranaires (fractions solubilisées et fractions non solubilisées). En effet, selon les substituants présents, notamment la longueur des groupes alkyles dans R¹, R², R³ et/ou R⁴, les calixarènes de formule (I) peuvent s'ajuster aux types de membranes et de protéines et permettre de réaliser une extraction et une solubilisation sélectives et efficaces.

Selon un mode de réalisation particulier de l'invention, le procédé de l'invention peut comprendre deux ou plusieurs étapes successives de mise en contact avec différents calixarènes de formule (I), ce qui permet l'extraction et l'isolation de protéines membranaires les unes par rapport aux autres.

En outre, de par leur structure notamment la nature des substituants X¹, X² et/ou X³, les calixarènes selon l'invention, peuvent former des micelles dont l'existence est modulable en fonction du pH. Cette propriété est particulièrement utile pour favoriser ou au contraire défavoriser la formation de micelles et ainsi maintenir une protéine membranaire en solution avec des détergents à des degrés différents d'organisation. En effet, l'absence de micelles favorise l'établissement de contacts protéine-protéine qui facilitent la formation de cristaux en solution.

Le procédé de l'invention permet d'extraire toute protéine membranaire, et avantageusement toute protéine membranaire présente dans une fraction membranaire biologique issue d'organisme procaryotique ou eucaryotique, sain ou altéré. Par exemple, dans le procédé selon l'invention, la protéine membranaire à extraire peut être dans une fraction de membrane plasmique issue d'organisme procaryotique ou eucaryotique, sain ou altéré. Par exemple, le procédé de l'invention permet d'extraire toute protéine membranaire polytopique. Par exemple, le procédé de l'invention permet d'extraire toute protéine membranaire intégrale.

Selon un mode de réalisation de l'invention, la protéine membranaire peut-être choisie dans le groupe comprenant les protéines de transport. On entend par « protéine de transport » au sens de la présente invention, une protéine membranaire dont le rôle est le transport de part et d'autre de la membrane de diverses substances (ions, stérols, macromolécules, etc.). On peut citer par exemple les transporteurs ABC (ATP-binding Cassette en anglais ou protéine à domaine de liaison à l'ATP en français), les récepteurs, les échangeurs, les canaux, etc.

Selon un mode de réalisation particulier de l'invention, la protéine de transport peut-être un transporteur ABC choisi dans le groupe comprenant la glycoprotéine P (Pgp/ABCB1) [1], MRP1/ABCC1 (multidrug résistance protein, transporteur ABC humain polytopique et intégrale) [2], BCRP/ABCG2 (breast cancer résistance protein, transporteur ABC humain polytopique et intégral) [3], BmrA (Bacterial multidrug resitance ATP, transporteur ABC procaryote polytopique et intégrale) [4].

De préférence, la protéine membranaire est une protéine choisie dans le groupe comprenant, comme protéines de transport, la glycoprotéine P (Pgp/ABCB1), MRP1/ABCC1, BCRP/ABCG2, et toutes protéines de cette classe. Plus particulièrement, la protéine de transport peut être un transporteur ABC, choisie dans le groupe comprenant BmrA.

L'étape de mise en contact d'une solution aqueuse comprenant la protéine membranaire à extraire avec au moins un calixarène de formule (I) peut être réalisé à un pH allant de 5.5 à 10, de préférence de 6 à 9.

Le procédé d'extraction peut être réalisé à une température allant de 0 à 100°C, de préférence de 4 à 25°C.

Le procédé d'extraction met en oeuvre une concentration de calixarène de formule (I) allant de 10⁻⁶ à 10⁻² M.

Le procédé de l'extraction selon l'invention peut étre réalisé avec des calixarènes en solution ou des calixarènes en agrégats colloïdaux du fait de leur propriété tensioactive.

Au sens de la présente invention, on entend par agrégat colloïdal des ensembles de quelques à quelques centaines de molécules de calixarène s'organisant en fonction des forces de répulsion vis-à-vis du solvant. Étant donné leur nature, les calixarènes de formule (I) sont capables de former des agrégats dans un milieu approprié comme par exemple dans l'eau, dans une solution aqueuse, dans un milieu isotonique ou dans un milieu biologique.

L'agrégat peut être choisi dans le groupe comprenant les micelles, les liposomes, les nanoparticules lipidiques. De préférence, les calixarènes sont sous forme de micelles.

Le terme micelle désigne est un agrégat sphéroïdal de molécules de calixarène de formule (I) qui s'organise en fonction du solvant utilisé. Par exemple dans l'eau ou un solvant aqueux, les extrémités lipophiles sont tournées vers l'intérieur de la micelle tandis que les extrémités hydrophiles forment l'interface de la micelle avec le solvant. Dans un solvant organique, par exemple de l'huile, l'arrangement est inversé.

Le terme liposome désigne des petites vésicules fabriquées artificiellement constituées notamment de lamelles de phospholipides, séparées les unes des autres par des compartiments aqueux. Ils peuvent avoir une structure très proche de celle des membranes cellulaires.

Dans le cadre de la présente invention, le terme nanoparticule signifie un assemblage de quelques centaines à quelques milliers de molécules de calixarène de formule (I), conduisant à un objet dont au moins l'une de ses dimensions est de taille nanométrique, par exemple entre 1 et 300 nm. Dans le procédé de l'invention, l'étape de mise en contact d'une suspension aqueuse comprenant la protéine membranaire à extraire avec le calixarène de formule (I) peut être éventuellement réalisée en présence d'au moins un co-soluté choisi dans le groupe comprenant,
i) les sels organiques et inorganiques choisi dans le groupe comprenant les sels pharmaceutiquement acceptables ;
ii) les petites molécules bioactives choisies dans le groupe des acides aminés, des vitamines, des lipides, des stéroïdes, des carbohydrates ou des métabolites ;
iii) les molécules bioactives oligomériques choisies dans le groupe des peptides, des oligonucléotides, des oligosaccharides ;
iv) les molécules biologiques polymériques choisies dans le groupe des protéines, polynucléotides et polysaccharides.

Selon un mode de réalisation de l'invention, l'étape de mise en contact peut être précédée par une étape dans laquelle :
- la protéine membranaire à extraire ou la fraction membranaire la contenant est solubilisée dans une solution tampon, et
- le calixarène de formule (I) est ajouté suivant les conditions de température, de pH et de concentrations décrites précédemment.

Selon un mode de réalisation de l'invention, l'étape de mise en contact peut être suivie par une étape de centrifugation, permettant de séparer les protéines membranaires complexées avec les calixarènes de formule (I) des protéines membranaires non complexées.

Les protéines membranaires complexées avec les calixarènes de formule (I) et les protéines membranaires non complexées peuvent être séparées par centrifugation, par exemple pendant 1 h, à 4° C et à une vitesse de 100 000xg. Les conditions de centrifugation dépendront de la nature de la protéine. L'homme du métier saura choisir les conditions les optimales de centrifugation, par exemple décrites dans [6].

D'autres avantages pourront encore apparaître à l'homme du métier à la lecture des exemples ci-dessous, illustrés par les figures annexées, donnés à titre illustratif.

### Brève description des figures

- La figure 1 représente la géométrie en forme de cône des calixarènes utilisés dans le procédé de l'invention. Le groupement polaire de tête est représenté en foncé et la chaîne aliphatique hydrophobe se retrouve au pied de la figure. Cette géométrie particulière rend possible la formation de micelles. Sur la figure 1, la région A correspond à la région chargée hydrophilique, la région B correspond à la plateforme du calix[4]arène et la région C correspond à une queue aliphatique en CₙH₂ₙ₊₁.

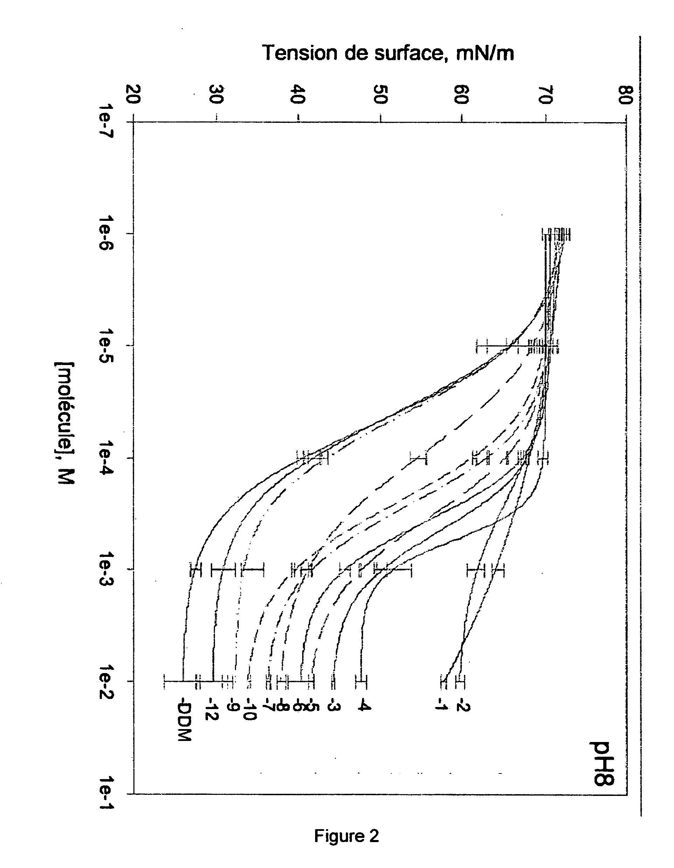

- La figure 2 représente les propriétés surfactantes (ou tensioactives) de molécules *p*(COOH)₃-Ar4-*o*(CₙH₂ₙ₊₁), n=1-12. L'effet des dérives de calix[4]arène, comportant des chaînes alkyle avec 1 à 12 atomes de carbone, sur la tension de surface de l'eau a été suivi en fonction de la concentration desdits dérivés (en molaire M ou mol/L) aux pH 6,0 (non exposé) et 8,0 (exposé). Les propriétés tensioactives du DDM (β-D-dodécyl maltopyranoside) sont également représentées sur cette figure. Chaque point correspond à la moyenne de trois valeurs.

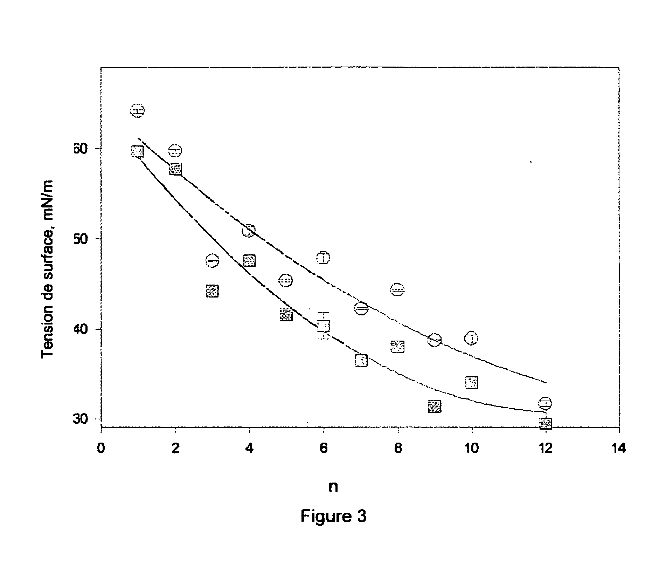

- La figure 3 représente le potentiel surfactant des molécules *p*(COOH)₃-Ar4-*o*(CₙH₂ₙ₊₁), en fonction de la longueur des chaînes alkyle (n = nombre d'atomes de carbone = 1-12) et le pH. L'effet des molécules comportant une chaîne alkyle ayant de 1 à 12 groupes méthylène (n, axe X dans la figure 3) sur la tension de surface de l'eau a été mesuré à une concentration de 10⁻²M, à pH 6,0 (triangles) et à pH 8,0 (carrés). Chaque point correspond à la moyenne de trois valeurs.

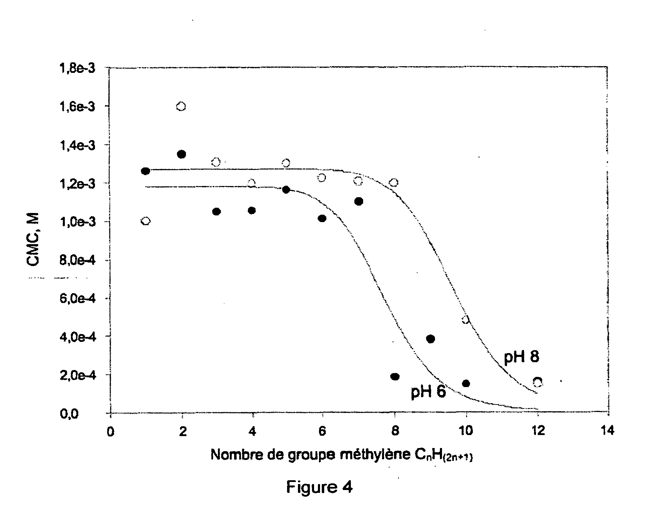

- La figure 4 représente la Concentration Micellaire Critique (CMC) de la série *p*(COOH)₃-Ar4-*o*(CₙH₂ₙ₊₁), n=1-12 en fonction du pH et la longueur des chaînes alkyle. L'effet sur la CMC des molécules ayant une chaîne alkyle avec 1 à 12 groupes méthylène (n, axe X sur la figure) est mesuré à une concentration de 10⁻² M, à pH 6,0 (cercles pleins) et 8,0 (cercles vides). Chaque point correspond à la moyenne de trois valeurs, estimées à partir des données de la figure 2.

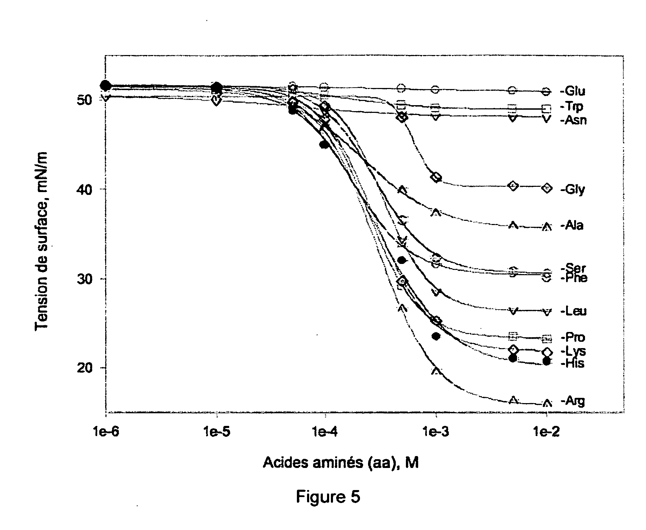

- La figure 5 représente la modulation du potentiel surfactant du composé *p*(COOH)₃-Ar4-*o*(C₇H₁₅) par les acides aminés (aa). Le composé *p*(COOH)₃-Ar4-*o*(C₇H₁₅) est présente à une concentration de 10⁻² M alors que celle des acides aminés varient comme indiqué. La tension de surface est mesurée au pH 8,0. Les acides aminés sont indiqués par leur code à trois lettre : Glu = acide glutamique, Trp = tryptophane, Asn = asparagine, Gly = glycine, Ala = alanine, Ser = sérine, Phe = phénylalanine, Leu = leucine, Pro = Proline, Lys = lysine, His = histidine, Arg = arginine. Chaque point correspond à la moyenne de trois valeurs.

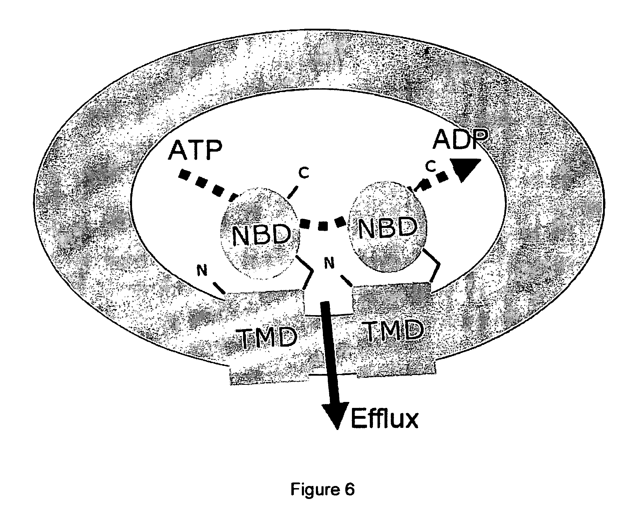

- La figure 6 représente le schéma topologique de BmrA exprimée dans une bactérie et insérée dans sa membrane plasmique. BmrA est une protéine homodimérique, chaque monomère étant constitué d'un domaine transmembranaire (TMD) lié à un domaine de liaison-nucléotide (nucléotide-binding domain (NBD)) [4, 5].

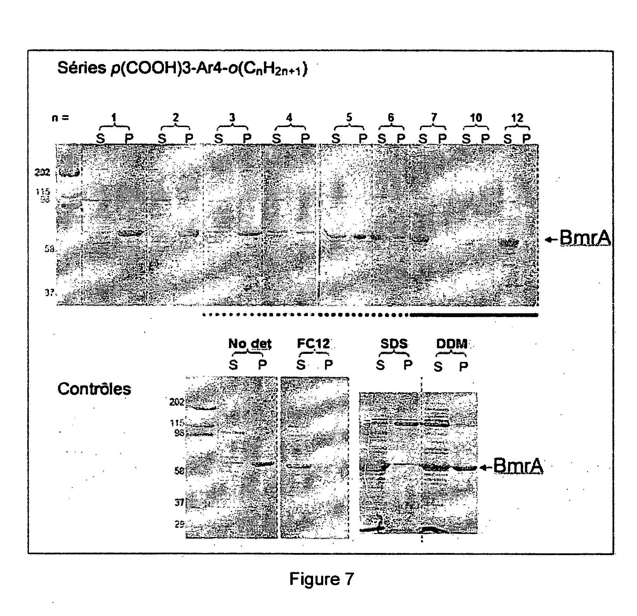

- La figure 7 représente les tests de solubilisation de membranes enrichies en BmrA avec les molécules *p*(COOH)₃-Ar4-*o*(CₙH₂ₙ₊₁) et d'autres détergents connus. Les fractions membranaires sont préparées comme indiqué dans l'exemple 11 et analysées après incubation soit avec des molécules *p*(COOH)₃-Ar4-*o*(CₙH₂ₙ₊₁), n= 1 to 12 comme indiqué dans la figure 7 soit avec des détergents connus (FC12, FosCholine 12: n-dodécylphosphocholine, détergent zwitterionique ; SDS : sodium dodécylsulfate, détergent anionique fort ; DDM : n-dodécyl-β-D-maltopyranoside, détergent non chargé), soit avec un tampon (No Det). Après séparation de la protéine solubilisée (S, surnageant) et non solubilisé (P, pellet ou culot) par centrifugation à 100 000xg, 1 h, 4°C, le contenu protéique est analysé par SDS PAGE. Les lignes pointillées grises et noires et la ligne noire servent d'échelle de l'efficacité des molécules à solubiliser. ) Les lignes « no det » signifient « sans détergent ».

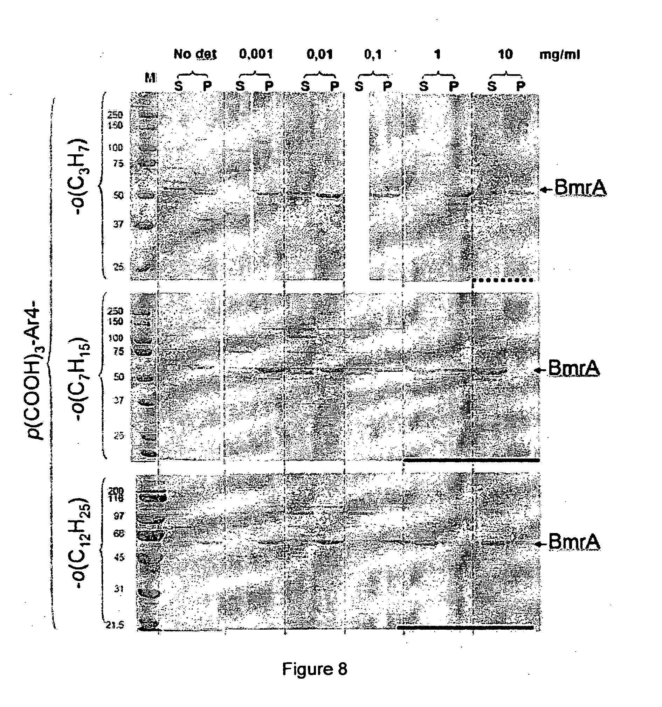

- La figure 8 représente l'effet de la concentration des molécules *p*(COOH)₃-Ar4-*o*(CₙH₂ₙ₊₁) sur la solubilisation de BmrA. Les conditions expérimentales sont celles de la figure 7, à l'exception des concentrations des molécules butyle, heptyle et dodécyle qui sont comme indiquées dans la figure, pour lesquelles la correspondance g/L <-> M est indiquée dans le tableau 2. Les lignes « no det » signifient « sans détergent ».

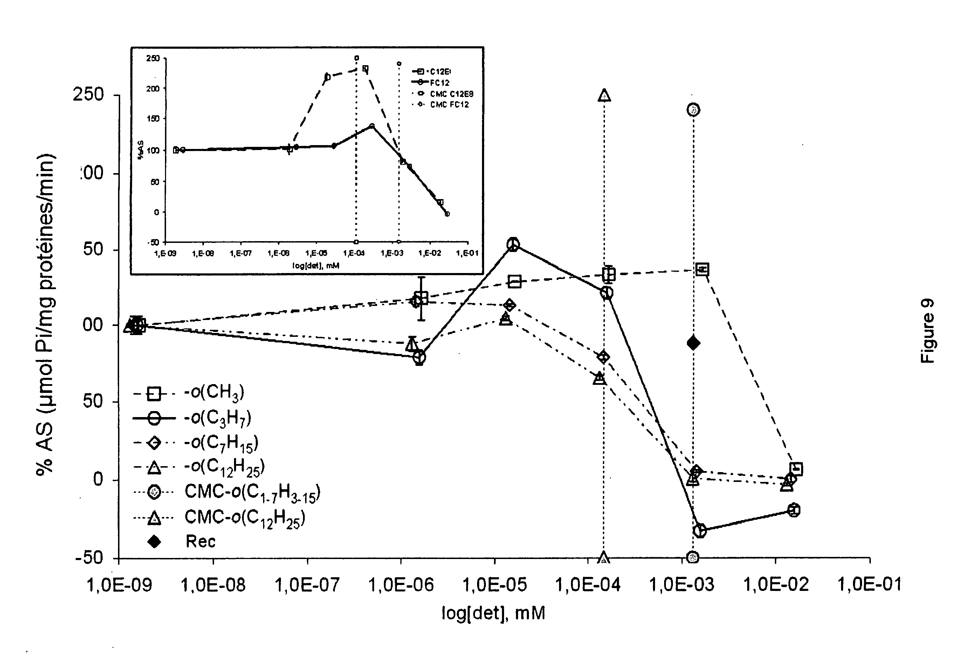

- La figure 9 représente l'effet de la solubilisation par des séries *p*(COOH)₃-Ar4-*o*(CₙH₂ₙ₊₁) sur l'activité ATPasique de BmrA. Les essais de solubilisation ont lieu comme dans la figure 8 et les essais d'activité ATPasique sont décrits dans l'exemple 11. Les lignes pointillées verticales représentent la CMC de chaque composé. Les losanges noirs correspondent à un essai dans lequel BmrA est solubilisée avec *p*(COOH)₃-Ar4-*o*(C₇H₁₅) puis reconstituée dans des lipides après élimination du détergent avec des biobeads (Bio-Rad) selon la procédure décrite dans Lenoir et al. [6]. L'axe des ordonnées représente l'activité spécifique en %, 100% d'activité correspondant à 0,25 µmol Pi produit/mg protéine/min.

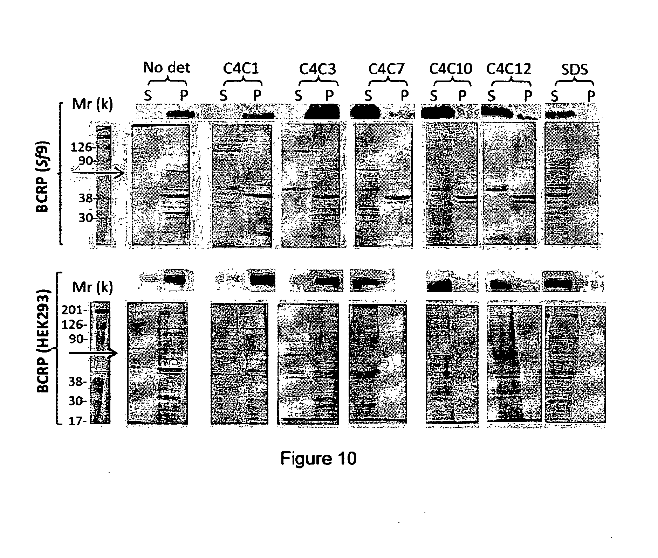

- La figure 10 représente l'extraction de la protéine BCRP (à préciser) à partir i) de cellules d'insecte Sf9 (en haut de la figure) et ii) de cellules humaines HEK293 (en bas de la figure). Ceci montre la capacité des calixarènes utilisés à extraire les protéines membranaires de differentes lignées de cellules.

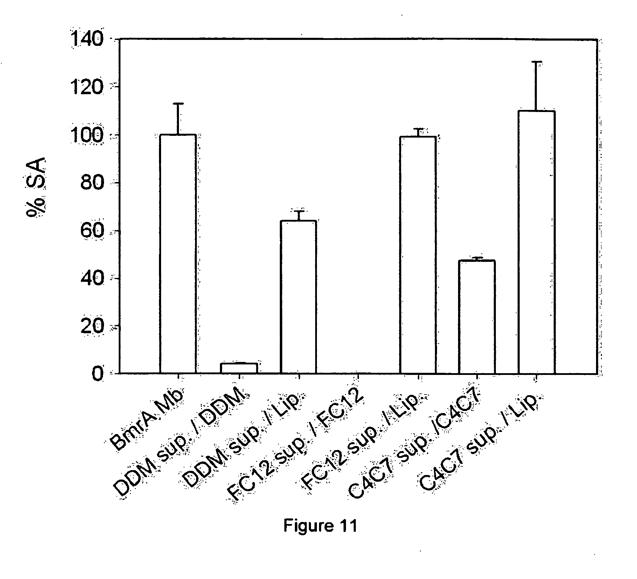

- La figure 11 représente l'activité de la protéine BmrA après extraction avec différents détergents, dont le β-D-dodécyl maltopyranoside (DDM), le Foscholine 12 (FC12) et le calix[4]arène-O-heptyloxy (C4C7). Le pourcentage d'activité ATPase spécifique sensible au vanadate (% SA ou % Specific ATPase Activity VO₄ sensitive) est évalué pour les différents cas suivants :
   - BmrA Mb : Activité du BmrA dans les membranes natives,
   - DDM sup./DDM : Activité du BmrA dans l'extrait de DDM,
   - DDM sup./Lip. : Activité du BmrA de l'extrait de DDM et reconstituté dans les liposomes,
   - FC12 sup.lFC12 : Activité du BmrA dans l'extrait de Foscholine 12,
   - FC12 sup./Lip.: Activité du BmrA de l'extrait de Foscholine 12 et reconstituté dans les liposomes,
   - C4C7 sup./FC12 : Activité du BmrA dans l'extrait de calixarène C4C7 (c'est-à-dire calix[4]arène-O-heptyloxy),
   - C4C7 sup./Lip. : Activité du BmrA de l'extrait de calixarène C4C7 et reconstituté dans les liposomes.

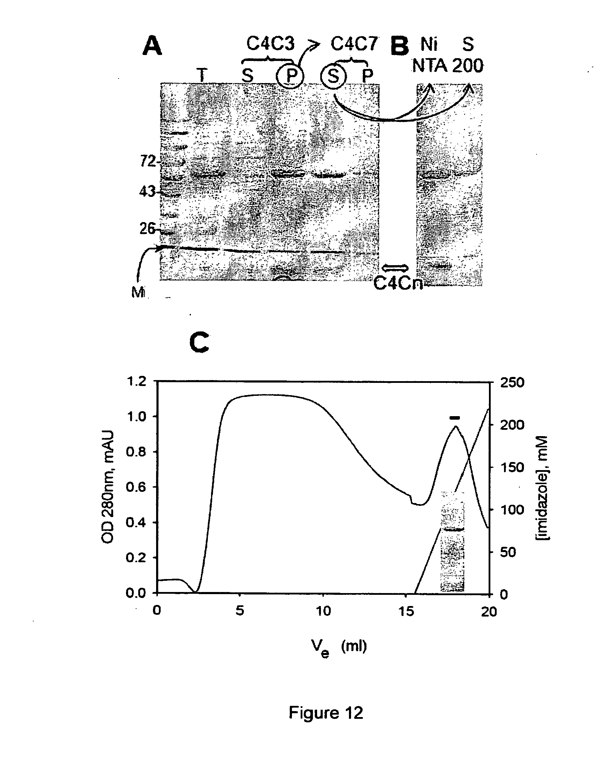

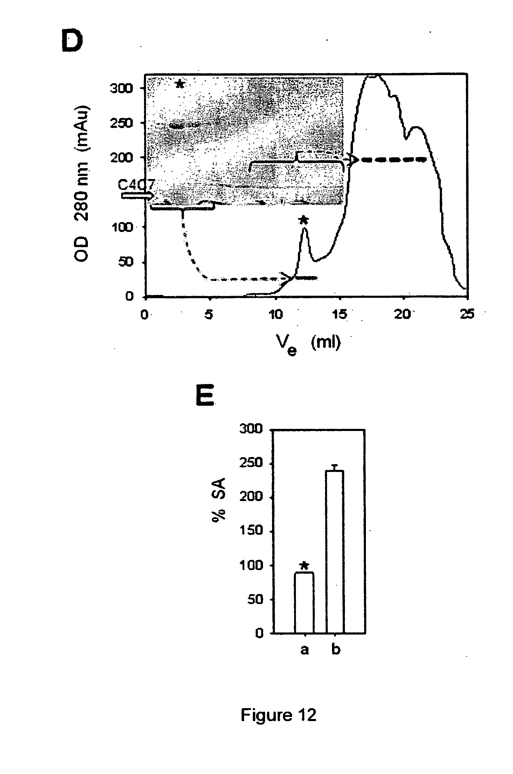

- La figure 12 résume les résultats d'une extraction en utilisant successivement les calixarènes C4C3 puis C4C7 (figure 12A) suivie d'une purification par chromatographie de gel filtration (figures 12B-E). La figure 12A représente le SDS-PAGE (SDS- polyacrylamide gel electrophoresis) des fractions solubilisée (S, surnageant) et non solubilisé (P, culot) après extractions successives par les calixarènes C4C3 puis C4C7, M représentant le front de migration. La figure 12C représente la chromatographie de nickel-affinité après extraction par le calixarène C4C7, en fonction du volume d'élution Vₑ en millilitre. La figure 12D représente la chromatographie de filtration sur gel en présence de FC12 après extraction par le calixarène C4C7, en fonction du volume d'élution Vₑ en millilitre. La figure 12E représente l'activité ATPase spécifique (sensible au vanadate) pour la fraction a (« S200 pool FC12 » ou fraction de BmrA soumis à chromatographie de filtration sur gel en présence de foscholine 12) et la fraction b (« S200 pool Lip. », la même fraction de BmrA soumise à filtration sur gel puis reconstituée en présence de liposomes).

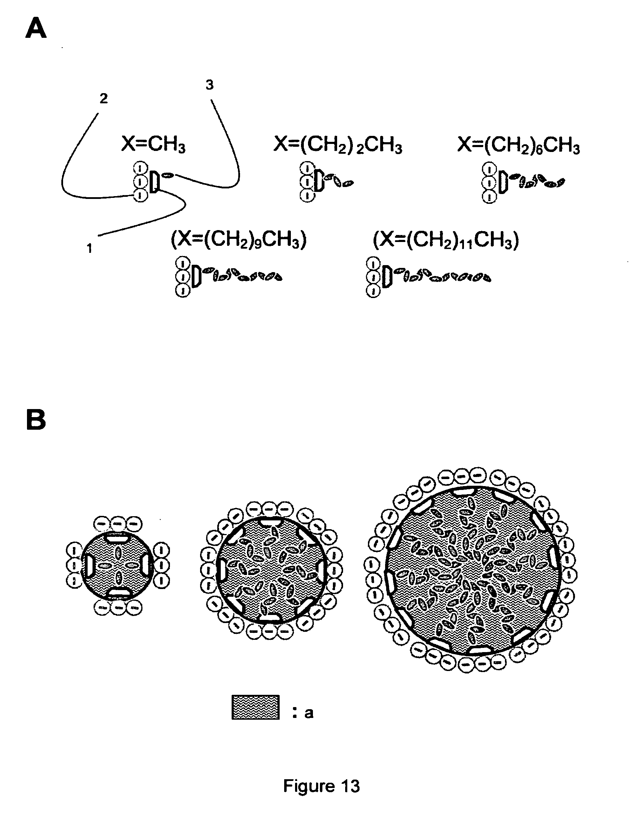

- La figure 13 représente différents calix[4]arènes (figure 14A) et des exemples d'organisation supramoléculaire de ces molécules en micelles ou amas supramoléculaires (figure 14B).

Dans la figure 13A, les calix[4]arènes représentés sont formés d'une couronne de 4 phénols (cycle, défini par le chiffre « 1 » sur la figure) portant sur une face les groupements carboxyliques (petit tiret dans un cercle, défini par le chiffre « 2 ») et sur l'autre face, une chaîne aliphatique CₙH₂ₙ₊₁, avec n= 1, 3, 7, 10 et 12 (petits bâtonnets, défini par le chiffre « 3 »).

Dans la figure 13B, les micelles (représentée par la zone « a ») formée par ces calixarènes exposent les fonctions chargées (petit tiret dans un cercle) à la surface avec une cohésion maintenue par les groupements hydrophobes (petits bâtonnets) regroupés à l'intérieur de l'amas.

### EXEMPLES

### Méthodes Générales

### Solvants et réactifs

Le dichlorométhane (CH₂Cl₂) et le toluène sont distillés, sous atmosphère d'azote, sur CaH₂, le tétrahydrofurane (THF) est distillé, sous atmosphère d'azote, sur sodium et benzophénone. Le diéthyl éther est distillé, sous atmosphère d'azote, sur CaH₂ et conservé à 0-4°C sous azote sur Tamis moléculaire 4Å. Les autres solvants utilisés proviennent du fournisseur Carlo-Erba.

### Résonance Magnétique Nucléaire (RMN)

Les spectres ¹H et ¹³C sont effectués sur un appareil Bruker AV500. Les déplacements chimiques (δ) des spectres RMN ¹H sont calibrés selon le témoin Tetraméthylsilane (TMS) ayant pour valeur de δ 0.00PPM Les δ des spectres de RMN ¹³C sont calibrés sur la valeur de référence du solvant. Les mesures sont effectuées à 25°C dans des tubes de 5 mm de diamètre. Les spectres sont effectués dans des solvants deutérés provenant du fournisseur Aldrich ou SDS.

### Chromatographie

Les chromatographies sur couche mince (CCM) sont réalisées sur des plaques d'aluminium « TLC Silica gel 60F₂₅₄ » de Merck. Les composés sont révélés sous lampe UV (Ultra-Violet) ou/et sont trempés dans le révélateur à l'acide-phosphomolybdique dans l'acide sulfurique et l'éthanol suivi d'un chauffage avec un décapeur thermique.

Les colonnes chromatographiques sont réalisées avec un gel de silice (Silica gel 60 (40-63 µm) de Merck.

### Spectrométrie de masse

Masse ESI : Les échantillons sont analysés sur un spectromètre Perkin Elmer Sciex API 300 dans des solvants de qualité « pour Analyse ».

### Mesure de la tension superficielle

Les propriétés tensioactives des calixarènes de formule (I) sont évaluées en goutte assise (en anglais « sitting drop ») de 1 ml à l'aide d'un appareil Kibron à micro-dépression et en utilisant une seringue en acier inoxydable et une balance de Wilhelmy. Les courbes obtenues sont traitées avec le logiciel Sigmaplot v11.

### Exemple 1 : Synthèse de 25-Butyloxycalix[4]arène

À une suspension de calix[4]arène (20 g, 0.47 mol) dans du DMF anhydre (943 ml), des solutions de CsF (8.49 g, 1.2 équivalents) et d'iodobutane (35,47 ml, 10 équivalents) ont été ajoutées. Le mélange réactionnel est agité à 40°C pendant 96 h. Le progrès de la réaction est suivi par CCM ou TLC (Chromatographie sur Couche Mince ou Thin Layer Chromatography). Une fois la réaction terminée, elle est arrêtée par ajout d'acide hydrochlorique 1M (250 ml). Le milieu réactionnel est extrait avec du CH₂Cl₂ (2 x 200 ml). Les phases organiques sont rassemblées, lavées avec de l'eau (2 x 250 ml) et séchées sur du MgSO₄. Après évaporation du solvant, le produit brut restant est repris avec un mélange CH₂Cl₂/MeOH (1:1). La solution est ensuite filtrée pour éliminer le calix[4]arène qui n'a pas réagi et purifié par chromatographie sur colonne (CH₂Cl₂/hexane 1:1).

Un solide blanc est obtenu avec un rendement de 68% par la procédure p.f. = 239°C ;
**¹H RMN** (CDCl₃) δ 1.14 (t, 3H, ²J_{H-H} = 7.1 Hz, Ar-OCH₂CH₂CH₂C*H₃*), 1.74 (m, 2H, Ar-O(CH₂)₂C***H**₂*CH₃), 2.18 (m, 2H, Ar-OCH₂CH₂CH₂CH₃), 3.48 (d, 4H, ²J_{H-H} = 13.3 Hz, Ar-C***H***₂-Ar), 4.17 (t, 2H, ²J_{H-H} = 7.0 Hz, Ar-OC***H**₂* (CH₂)₂CH₃), 4.30 (d, 2H, ²J_{H-H} = 13.6 Hz, Ar-CH₂-Ar), 4.37 (d, 2H, ²J_{H-H} = 12.9 Hz, Ar-C***H₂***-Ar), 6.69-7.07 (m, 12H, Ar-***H***), 9.46 (s, 2H, Ar-O***H***), 9.77 (s, 1 H, Ar-O***H***).
**¹³C RMN** (CDCl₃) δ 14.2 (Ar-O(C***H₂***)₃CH₃), 19.2 (Ar-O(***C***H₂)₂CH₂CH₃), 19.7 (Ar-OCH₂CH₂***C***H₂CH₃), 31.4 and 32.3 (Ar-CH₂-Ar); 77.1 (Ar-O***C***H₂(CH₂)₂CH₃), 120.8; 121.8; 121.7; 126.1; 128.3; 128.7; 129.3 (Ar), 149.1 and 150.6 (Ar***C***-OH), 151.5 (ArC-O(CH₂)₃CH₃).
ES spectre de masse (CHCl₃) m/z: 481.2 [M+H]⁺, 503.3 [M+Na]⁺, 519.2 [M+K]⁺.

### Exemple 2 : Synthèse de 25-Dodécyloxycalix[4]arène

À une suspension de calix[4]arène (20 g, 0.47 mol) dans du DMF anhydre (943 ml), des solutions de CsF (8.49 g, 1.2 équivalents) et d'iodododécane (35.47 ml, 10 équvalents) ont été ajoutées. Le mélange réactionnel est agité à 40°C pendant 96 h. Le progrès de la reaction est suivi par TLC. Une fois la reaction terminée, elle est arrêtée par ajout d'acide hydrochlorique 1M (250 ml). Le milieu réactionnel est extrait avec du CH₂Cl₂ (2 x 200 ml). Les phases organiques sont rassemblées, lavées avec de l'eau (2 x 250 ml) et séchées sur du MgSO₄. Après évaporation du solvant, le produit brut restant est repris avec un mélange CH₂Cl₂/MeOH (1:1). La solution est ensuite filtrée pour éliminer le calix[4]arène qui n'a pas réagi et purifiée par chromatographie sur colonne (CH₂Cl₂/hexane 1:1).

Un solide blanc est obtenu avec un rendement de 48%, p.f. = 235°C; **¹H RMN** (CDCl₃) δ 1.01 (t, 3H, ³J_{H-H}= 7.0 Hz, Ar-O(CH₂)₁₁C***H***₃), 1.42 (m, 4H, Ar-O(CH₂)₈C*H*₂C*H*₂CH₃), 1.49 (m, 4H, Ar-O(CH₂)₇C***H***₂C***H***₂(CH₂)₂CH₃), 1.53 (m, 4H, Ar-O(CH₂)₅CH₂CH₂(C***H***₂)₄C***H***₃), 1.62 (m, 4H, Ar-O(CH₂)₃C***H***₂C***H***₂(CH₂)₆CH₃), 1.80 (m, 2H, Ar-O(C***H***₂)₂CH₂(CH₂)₈CH₃), 2.29 (m, 2H, Ar-OC***H***₂CH₂(CH₂)₉CH₃), 3.57 (d, 4H, ²J_{H-H} = 13.3 Hz, Ar-C***H***₂-Ar), 4.26 (t, 2H, ²J_{H-H} = 6.8 Hz, Ar-OC***H***₂(CH₂)₁₀CH₃), 4.39 (d, 2H, ²J_{H-H}= 13.1 Hz, Ar-C***H***₂-Ar), 4.48 (d, 2H, ²J_{H-H}= 13.1 Hz, Ar-C***H***₂-Ar), 6.77-7.18 (m, 12H, Ar-***H***), 9.57 (s, 2H, Ar-O***H***), 9.88 (s, 1 H, Ar-O***H***).
**¹³C NMR** (CDCl₃) δ 14.3 (Ar-O(CH₂)₁₁***C***H₃), 22.9 (Ar-O(CH₂)₁₀***C***H₂CH₃), 26.1(Ar- O(CH₂)₉***C***H₂CH₂CH₃), 29.6 (Ar-O(CH₂)₈***C***H₂(CH₂)₂CH₃), 29.7 (Ar-O(CH₂)₇***C***H₂(CH₂)₃CH₃), 29.8 (Ar-O(CH₂)₇***C***H₂(CH₂)₄CH₃), 29.8 (Ar-O(CH₂)₅***C***H₂(CH₂)₅CH₃), 29.9 (Ar-O(CH₂)₄***C***H₂(CH₂)₆CH₃), 30.1 (Ar-O(CH₂)₃***C***H₂(CH₂)₇CH₃), 31.6 (Ar-O(CH₂)₂***C***H₂(CH₂)₈CH₃), 31.8 (Ar-OCH₂CH₂(***C***H₂)₈CH₃), 32.1 and 32.2 (Ar-***C***H₂-Ar), 77.7 (Ar-O***C***H₂(CH₂)₁₀CH₃) 121.1; 122.1; 122.4; 126.2; 128.4; 128.6; 128.9; 129.1; 129.5; 134.4 (Ar), 149.4 and 151.0 (ArC-OH), 151.6 (ArC-O(CH₂)₁₁CH₃).
ES spectre de masse (CHCl₃) m/z: 593.2 [M+H]⁺, 615.2 [M+Na]⁺, 631.3 [M+K]⁺.

### Exemple 3 : Synthèse de 5,11,17 Tris-[(Carboxylato)méthyl] 25-monométhyloxy 26,27,28 tris-hydroxycalix[4]arène - composé 5a

Dans un ballon sont placés 5g de tris-[(cyano)méthyl] monométhyloxy26,27,28 tris-hydroxycalix[4]arène dans 50 ml de EtOH. 50 ml de KOH 3M sont ajoutés. Le mélange réactionnel est chauffé à reflux pendant 72h. La solution est refroidie, traitée avec 500ml d'eau glacée, acidifié avec une solution HCl 12N. Le produit jaune est récupéré par filtration et placé puis porté à reflux pendant 72h dans 50 ml de MeOH et 4.5 g de NaOH dans 50 ml d'eau. La solution est ensuite refroidie, traitée avec 250ml d'eau glacée, acidifiée avec du HCl, filtrée, lavée à l'eau pour fournir le 5,11,17 tris-[(carboxy)méthyl] 25-monométhyloxycalix[4]arène sous forme d'une poudre blanc cassé à jaune avec un rendement de 72%. p.f. = 221 °C,
**¹H RMN** (DMSO) δ 3.24 (s, 2H, Ar-C*H*₂COOH), 3.31 (s, 4H, Ar-C*H*₂COOH),_3,39(d,_2H, ²J_{H-H} = Hz, Ar=C*H*₂-Ar), 3.48 (d, 2H, ²J_{H-H} = 13.2 Hz, Ar-C*H*₂-Ar), 4.01 (s, 3H, Ar-OC*H*₃), 4.18 (d, 2H, ²J_{H-H} = 13.2 Hz, Ar-C*H₂*-Ar), 4.25 (d, 2H, ²J_{H-H} = 13.2 Hz, Ar-C*H*₂-Ar), 6.89-7.16 (m, 9H, Ar-H), 8.79 (s, 2H, Ar-OH), 9.50 (s, 1 H, Ar-OH), 12.07 (s, 3H, Ar-CH₂-COO*H*), **¹³C RMN** (DMSO) δ 21.1 (Ar-CH₂-COOH), 29.1 and 30.9 (Ar-CH₂-Ar), 66.1 (Ar-OCH₃), 142.7; 143.1; 147.4; 148.4 (Ar), 150.3 and 153.5 (ArC-OH), 154.1 (ArGOCH₃), 168.4 and 169.3 (Ar-CH₂-COOH).
ES spectre de masse (DMSO) m/z: 613.1 [M+H]⁺, 635.1 [M+Na]⁺, 611.3 [M-H]⁻.

### Exemple 4 : Synthèse de 5,11,17 Tris-((Carboxylato)méthyl] 25-monobutyloxy 26,27,28 tris-hydroxycalix[4]arène - composé 5b

Dans un ballon sont placés 5g de tris-[(cyano)méthyl] monobutyloxy26,27,28 tris-hydroxycalix[4]arène dans 50 ml de EtOH. 50 ml de KOH 3M sont ajoutés. Le mélange réactionnel est chauffé à reflux pendant 72h. La solution est refroidie, traitée avec 500ml d'eau glacée, acidifié avec une solution de HCl 12N. Le produit jaune est récupéré par filtration puis porté à reflux pendant 72 h dans 50 ml MeOH et 4.5 g of NaOH dans 50 ml d'eau. La solution est refroidie, traitée avec 250ml d'eau glacée, acidifiée avec du HCl, filtrée, et lavée avec de l'eau pour fournir le 5,11,17 tris-[(carboxy)méthyl] 25-monobutyloxycalix[4]arène sous forme d'une poudre blanc cassé à jaune avec un rendement de 75%.
p.f. = 223°C,
**¹H RMN** (DMSO) δ 1.07 (t, 3H, ²J_{H-H} = 6.9 Hz, Ar-O(CH₂)₃C***H₃***), 1.69 (m, 2H, Ar-O(CH₂)₂C***H₂***CH₃), 2.01 (m, 2H, Ar-OCH₂C***H₂***CH₂CH₃), 3.17 (s, 2H, Ar-C***H₂***COOH), 3.31 (s, 4H, Ar-C***H₂***COOH), 3.38 (d, 2H, ²J_{H-H} =13.1 Hz, Ar-C***H₂***-Ar), 3.48 (d, 2H, 2H, ²J_{H-H} = 13.1 Hz, Ar-C***H₂***-Ar), 3.57 (t, 2H, ²J_{H-H} = Hz, Ar-OC***H₂***(CH₂)₂CH₃), 4.11 (d, 2H, ²J_{H-H} = 13.1 Hz, Ar-C***H₂***-Ar), 4.20 (d, 2H, ²J_{H-H} = 13.1 Hz, Ar-C***H₂***-Ar), 6.82-7.12 (m, 9H, Ar-***H***), 8.87 (s, 2H,
**Ar-O*H*), 9.56 (s, 1H, Ar-O*H*),**
**¹³C RMN** (DMSO) δ 13.8 (Ar-O(CH₂)₃***C***H₃), 18.6 (Ar-O(CH₂)₂***C***H₂CH₃), 22.1 (Ar-***C***H₂-COOH), 30.3 (Ar-OCH₂***C***H₂CH₂CH₃), 30.6 and 31.4 (Ar-***C***H₂-Ar), 76.6 (Ar-O***C***H₂(CH₂)₂CH₃), 125.7; 127.6; 128.7, 130.1; 134.0 (Ar), 147.8 and 150.0 (Ar***C***-OH), 151.9 (Ar***C***-O(CH₂)₃CH₃), 172.9 (Ar-CH₂-***C***OOH).
ES spectre de masse (DMSO) m/z: 677.1 [M+Na]⁺, 653.3 [M-H]⁻.

### Exemple 5 : Synthèse de 5,11,17 Tris-[(Carboxylato)méthyl] 25-monohexyloxy 26,27,28 tris-hydroxycalix[4]arène - composé 5c

Dans un ballon sont placés 5g de tris-[(cyano)méthyl] monohexyloxy26,27,28 tris-hydroxycalix[4]arène dans 50 ml de EtOH. 50 ml de KOH 3M sont ajoutés. Le mélange réactionnel est chauffé à reflux pendant 72h. La solution est refroidie, traitée avec 500ml d'eau glacée, acidifié avec une solution de HCl 12M.

Le produit jaune est récupéré par filtration puis porté à reflux pendant 72 h dans 50 ml MeOH et 4.5 g of NaOH dans 50 ml d'eau. La solution est refroidie, traitée avec 250ml d'eau glacée, acidifiée avec du HCl, filtrée, et lavée avec de l'eau pour fournir le 5,11,17 Tris-[(carboxy)méthyl] 25-monohexyloxycalix[4]arène sous forme d'un solide jaune avec un rendement de 69%. m.p = 222°C,
**¹H RMN** (DMSO) δ 0.95 (t, 3H, ²J_{H-H} = 7.1 Hz, Ar-O(CH₂)₅C***H***₃), 1.42 (m, 4H, Ar-O(CH₂)₃C***H***₂C***H***₂CH₃), 1.67 (m, 2H, Ar-O(CH₂)₂C***H***₂(CH₂)₂CH₃), 2.02 (m, 2H, Ar-CH₂C***H***₂(CH₂)₃CH₃), 3.16 (s, 2H, Ar-C***H***₂-COOH), 3.31 (s, 4H, Ar-C***H***₂-COOH), 3.38 (d, 2H, ²J_{H-H} = 12.9 Hz, Ar-C***H***₂-Ar), 3.50 (d, 2H, ²J_{H-H} = 12.9 Hz, Ar-C***H***₂-Ar), 4.04 (t, 2H, ²J_{H-H} = 7.2 Hz, Ar-OC***H***₂(CH₂)₄CH₃), 4.15 (d, 2H, ²J_{H-H} = 12.9 Hz, Ar-C***H***₂-Ar), 4.20 (d, 2H, ²J_{H-H} = 12.9 Hz, Ar-C***H***₂-Ar). 6.85-7.18 (m, 9H, Ar-***H***), 8.89 (s, 2H, Ar-O***H***), 9.53 (s, 1H, Ar-O***H***), **¹³C NMR** (DMSO) δ 14.0 (Ar-O(CH₂)₅***C***H₃), 22.3 (Ar-***C***H₂-COOH), 24.8 (Ar-O(CH₂)₄***C***H₂CH₃), 29.3 (Ar-O(CH₂)₃***C***H₂CH₂CH₃), 29.7 (Ar-O(CH₂)₂***C***H₂(CH₂)₂CH₃), 30.3 (Ar-OCH₂***C***H₂(CH₂)₃CH₃), 30.6 and 31.7 (Ar-***C***H₂-Ar), 77.2 (Ar-O***C***H₂(CH₂)₄CH₃), 123.2; 124.2; 125.4; 126.9; 127.9; 129.6; 134.0 (Ar), 147.8 and 150.0 (Ar***C***-OH), 151.9 (Ar***C***-O(CH₂)₅CH₃).
ES spectre de masse m/z: 683.1 [M+H]⁺, 681.5 [M-H]⁻.

### Exemple 6 : Synthèse de 5,11,17 Tris-[(Carboxylato)méthyl] 25-monododécyl 26,27,28 tris-hydroxycalix[4]arène - composé 5d

Dans un ballon sont placés 5g de tris-[(cyano)méthyl] monododécyloxy26,27,28 tris-hydroxycalix[4]arène dans 50 ml de EtOH. 50 ml de KOH 3M sont ajoutés. Le mélange réactionnel est chauffé à reflux pendant 72h. La solution est refroidie, traitée avec 500ml d'eau glacée, acidifié avec une solution de HCl 12M.

Le produit jaune est récupéré par filtration puis porté à reflux pendant 72 h dans 50 ml MeOH et 4.5 g of NaOH dans 50 ml d'eau. La solution est refroidie, traitée avec 250ml d'eau glacée, acidifiée avec du HCl, filtrée, et lavée avec de l'eau pour fournir le 5,11,17 tris-[(carboxy)méthyl] 25-monododécyloxycalix[4]arène sous forme d'un solide jaune avec un rendement de 71%,
p.f. = 210°C,
**¹H RMN** (DMSO) δ 0.81 (t, 3H, ²J_{H-H} = 7.0 Hz, Ar-O(CH₂)₁₁CH₃), 1.01 (m, 4H, **A**r-O(CH₂)₉CH₂C***H***₂C***H***₃), 1.03 (m, 4H, **Ar**-O(CH₂)₇C***H₂***C***H₂***C***H₂***)*₂*CH₃), 1.10 (m, 4H, Ar-O(CH₂)₅C***H***₂C***H***₂CH₂)₄CH₃), 1.25 (m, 2H, Ar-O(CH₂)₄C***H*₂**(CH₂)₅CH₃), 1.44 (m, 2H, **Ar**-O(CH₂)₃C***H₂***(CH₂)₆CH₃), 1.67 (m, 2H, Ar-O(CH₂)₂C***H₂***(CH₂)₂CH₃), 2.01 (m, 2H, Ar-OCH₂CH₂(CH₂)₈CH₃), 3.18 (s, 2H, Ar-C***H₂***COOH), 3.30 (s, 4H, Ar-C***H₂***COOH), 3.35 (d, 2H, ²J_{H-H} = 13.2 Hz, Ar-C***H₂***-Ar), 3.47 (d, 2H, ²J_{H-H} = 13.2 Hz, Ar-C***H₂***-Ar), 4.01 (t, 2H, 2JH-H = 7.1 Hz, Ar-OCH₂(CH₂)₁₀CH₃), 4.13 (d, 2H, ²J_{H-H} = 13.2 Hz, Ar-C***H₂***Ar), 4.17 (d, 2H, ²J_{H-H} = 13.2 Hz, Ar -C***H₂***-Ar), 6.83-7.12 (m, 9H, Ar-H), 8.91 (s, 2H, Ar-O***H***), 9.53 (s, 1H, Ar-O***H***),
¹³C **RMN** (DMSO) δ 13.8 (Ar-O(CH₂)₁₁CH₃), 21.2 (Ar-CH₂-COOH), 24.6 (Ar-O(CH₂)₁₀CH₂CH₃), 24.8 (Ar-O(CH₂)₉CH₂CH₂CH₃), 28.7 (Ar-O(CH₂)₈CH₂(CH₂)₂CH₃), 28.8 (Ar-O(CH₂)₇CH₂(CH₂)₃CH₃), 29.1 (Ar-O(CH₂)₆CH₂(CH₂)₄CH₃), 29.4 (Ar-O(CH₂)₅CH₂(CH₂)₅CH₃), 29.6 (Ar-O(CH₂)₄CH₂(CH₂)₆CH₃), 29.8 (Ar-O(CH₂)₃CH₂(CH₂)₇CH₃), 29.9 (Ar-O(CH₂)₂CH₂(CH₂)₈CH₃), 30.2 (Ar-OCH₂CH₂(CH₂)₉CH₃), 30.5 and 31.6 (Ar-CH₂-Ar), 77.8 (Ar-OCH₂(CH₂)₁₀CH₃), 123.1; 124.3; 124.6; 127.2; 128.4; 129.7; 134.1 (Ar), 147.1 and 150.3 (ArC-OH), 151.4 (ArC-O(CH₂)₁₁CH₃), 170.3 (Ar-CH₂-COOH).
ES spectre de masse m/z: 767.2 [M+H]⁺, 765.1 [M-H]-.

### Exemple 7 : Propriétés surfactantes -1

La figure 2 montre l'effet surfactant des molécules p(COOH)₃-Ar4-o(CₙH₂ₙ₊₁), n=1-12 atomes de carbone. Comme montré, la capacité à diminuer la tension de surface de l'eau, un effet typique d'un surfactant, augmente avec le nombre d'atomes de carbones sur la chaîne aliphatique. Les molécules de calixarène ayant une chaîne aliphatique plus courte modifient peu la tension de surface de l'eau, ce qui signifie que leurs propriétés détergentes sont limitées. Par contre, les molécules comportant une chaine alkyle plus longue (n= 3 à 12) se comportent comme des détergents.

Comme attendu, le potentiel du surfactant augmente avec la longueur de la chaîne alkyle, et la molécule p(COOH)₃-Ar4-o(C₁₂H₂₅) montre des propriétés tensioactives proches à celles de DDM (β-D-dodécyl maltopyranoside), un détergent non ionique classiquement utilise pour solubiliser la membrane des protéines.

Cet exemple illustre la première propriété des composés calixarènes de l'invention, c'est-à-dire que les molécules de calixarènes p(COOH)₃-Ar4-o(CₙH₂₊₁), n=3-12 se comportent comme des surfactants.

Comme montré dans la figure 3, le pH module le potentiel des molécules sur la tension de surface. A pH 6 et 8, la tension de surface augmente régulièrement avec la longueur des chaînes alkyle atteignant un plateau qui correspond à la moitié de la surface de tension initial (n = 12 à pH 8.0).

Ces effets illustrent une propriété de l'invention qui est que le simple changement du pH permet de moduler la tension de surface du milieu en présence des calixarènes de l'invention. Cet effet peut être réduit ou amplifié en fonction de la longueur de la chaîne alkyle des calixarènes de l'invention.

### Exemple 8 : Propriétés surfactantes - 2

Comme montré dans la figure 4, les calixarènes p(COOH)₃-Ar4-o(CₙH₂ₙ₊₁), n=1-12 se comportent comme des détergents avec une concentration micellaire critique (CMC) pouvant aller de 0,15 à 1,5 mM. Par concentration micellaire critique (CMC), il est entendu la concentration au delà de laquelle la concentration des molécules détergentes n'augmente plus car lesdites molécules sont impliquées dans des micelles (figure 13). Les molécules comportant une chaîne alkyle avec un nombre de groupes méthylène inférieur à 8 montrent une CMC de l'ordre du mM qui ne dépend pas du pH. Au contraire, lorsque les chaînes alkyle incluent plus de 8 groupes méthylène, la CMC résultante diminue sensiblement à 0,15mM lorsque le pH augmente de 6 à 8.

Cet exemple illustre une propriété importante des calixarènes de la série *p*(COOH)₃-Ar4-o(CnH₂ₙ₊₁), n=1-12 dont la sensibilité au pH dépend de la longueur de la chaîne alkyle. Cette propriété est particulièrement importante pour les calixarènes *p*(COOH)₃-Ar4-O(CₙH₂₊₁), n=9-12 qui peuvent être utilisés soit à un pH acide soit à un pH basique, pour favoriser ou défavorise la formation des micelles. Cette propriété est typiquement pour faciliter l'élimination du détergent sans en augmenter la concentration pendant l'étape de concentration de la membrane de la protéine solubilisée.

### Exemple 9 : Interaction de p(COOH)₃-Ar4-o(CₙH₂ₙ₊₁), avec les acides aminés

La figure 5 illustre la propriété unique des calixarènes de la série *p*(COOH)₃-Ar4-*o*(CₙH₂ₙ₋₁), exemplifié avec n=7, à former des complexes avec les acides aminés, modulant la tension de surface du milieu. Aucun effet n'est observé avec les acides aminés tels que l'acide glutamique, ou son dérivé aminé, l'asparagine. Un effet modéré est observé avec la glycine. Cet effet est amplifié avec l'hydrophobicité (alanine, phénylalanine, leucine) et devient très prononcé avec les acides aminés chargés positivement tels que l'histidine, la lysine et l'arginine.

Cet exemple illustre une propriété importante de la molécule *p*(COOH)₃-Ar4-*o*(C₇H₁₅) (et d'autres), avec lequel le potentiel surfactant peut être augmenté facilement en ajoutant des acides aminés chargés positivement. Les complexes résultants sont proches des composés zwittérioniques.

### Exemple 10 : Taille des agrégats

Le tableau 1 indique l'état d'agrégation des molécules *p*(COOH)₃-Ar4-*o*(CnH₂ₙ₊₁).

**Tableau 1. L'état d'agrégation des molécules p(COOH)₃-Ar4-o(CₙH₂ₙ₊₁)**

| Diamètre apparent (nm) | | | | |
|---|---|---|---|---|
| composé | pH 2 | pH4 | pH 6 | pH 8 |
| | | | 22 | |
| **5a** | 950 | 340 | | 270 |
| | | | 260 | |
| | | 550 | | 270 |
| **5b** | 500 | | 145 | |
| | | 90 | | 25 |
| | | | | 210 |
| **5c** | 850 | 210 | 400 | |
| | | | | 6 |
| | | 890 | | |
| | | | | 730 |
| **5d** | 880 | 100 | 190 | |
| | | | | 8 |
| | | 40 | | |

Comme illustré dans le tableau 1, tous les systèmes observés forment des agrégats de type micellaire.

### Les effets biologiques des molécules p(COOH)₃-Ar4-o(CₙH₂ₙ₊₁)

Les séries *p*(COOH)₃-Ar4-*o*(CₙH₂ₙ₊₁) ont fait l'objet d'essais pour la solubilisation de BmrA, une protéine membranaire appartenant aux transporteurs ABC et qui protège les cellules contre l'efflux et la fuite des xenobiotiques vers l'extérieur de la cellule [Chami 2002 JMB 315 1075 ; Orelle 2003 JBC 278-47 47002].

Comme schématisé dans la figure 6, BmrA est une protéine homodimérique insérée dans la membrane plasma de la bactérie. Cette protéine efflue des xénobiotiquesvia l'hydrolyse d'ATP, une activité enzymatique qui dépend du repliement correct du transporteur et qui requiert des lipides [7]. Une telle activité est utilisée pour vérifier l'intégrité fonctionnelle de la protéine au cours de la procédure de solubilisation.

### Exemple 11 : Capacité de solubilisation des membranes biologiques des calixarènes de l'invention

Les bactéries compétentes C41 BL21 (DE3) *E*. *coli* (*Escherichia coli*) ont été transformées avec le plasmide pET15b-BMRA portant le gène codant pour BmrA et placées sous le contrôle d'un promoteur inductible et un gène codant pour la protéine conférant de la résistance à l'ampicilline. Un clone positif a été cultivé dans un milieu liquide à 37°C, 200 rpm en présence d'ampicilline jusqu'à une phase de croissance mi-exponentielle. L'expression de BmrA est induite par addition de 1 mM d'isopropyl thio β-galactoside et continuée à 25°C pendant 4 h. Les bactéries ont été cueillies par centrifugation, cassées dans une presse de French (French press), et la fraction membranaire a été isolée par une série d'étapes de centrifugation à vitesses faible et élevée. Le contenu des protéines a été évalué par colorimétrie [8] et fixé à 20 mg/ml (20 mM Tris-Cl pH 8,0, 200 ml NaCl, 300 mM sucrose) avant congélation et conservation dans de l'azote liquide. Avant usage, la fraction membranaire est ensuite rapidement dégelée dans l'eau et maintenue à 4°C dans de la glace.

La solubilisation est effectuée à 4°C (ou à température ambiante - 23°C- quand indiqué, en diluant dans le même tampon la fraction membranaire à une concentration finale de 2 mg protéines/ml, puis en ajoutant les détergents et les molécules testées à 10 mg/ml (1%) ou moins quand indiqué, suivi d'une incubation de 2 h à 4°C. Les fractions de protéines solubilisées ou non solubilisées sont séparées par une étape de centrifugation de 200 000xg pendant 1 h à 10°C. Chaque culot de centrifugation est suspendu dans le volume initial et chargé, ensemble avec le surnageant sur une électrophorèse du type Laemmli gel polyacrylamide (SDS PAGE) [9] et comme décrit par exemple dans [7] pour analyser leur contenu en protéines. BmrA migre dans de telles conditions à environ 70 000 daltons.

L'activité ATPasique est mesurée en évaluant le contenu en phosphate inorganique (Pi) après 5 minutes d'incubation à 37°C. La fraction de Pi produite par d'autres activités ATPasique est déduite en mesurant la même activité en présence de 1 mM d'ortho vanadate qui inhibe l'activité ATPase de BmrA (par exemple comme décrit dans [10].

Comme montré dans la figure 7, BmrA est progressivement retrouvée dans le surnageant, en fonction de la longueur de la chaîne alkyle des molécules *p*(COOH)3-Ar4-*o*(CₙH₂ₙ₊₁). Aucune solubilisation n'a lieu avec les molécules les plus courtes (n=1,2) donnant un schéma comparable à celui obtenu avec aucun détergent (lignes « no det »). La solubilisation augmente progressivement avec n=3,4, et est plus prononcée avec n=5 et 6 conduisant au même schéma que celui obtenu avec DDM. La solubilisation paraît complète pour n ≥ 7, comme obtenue avec FC12 ou SDS. En conséquence, ces molécules solubilisent efficacement les protéines membranaires.

Ceci est confirmé avec les données de la figure 8, qui montrent que la solubilisation a lieu à une concentration en molécules ≥ CMC, environ 1.5 mM pour le composé heptyle (1g/L=1.6mg/ml) alors que ce n'est pas le cas pour le composé dodécyle étant donné qu'à une CMC de 0,15 mM (correspondant à 0.1g/L), aucune solubilisation n'a lieu, probablement à cause d'un rapport détergent / protéine trop faible.

Le tableau 2 indique la correspondance g/L en M pour les molécules testées.

**Tableau 2 : Poids moléculaires (PM), concentrations micellaires critiques (CMC) et correspondance entre la masse et les concentrations molaires des molécules testées**

| ***p*(COOH)₃-Ar4-** | **[composés], M** | | | | | | |
|---|---|---|---|---|---|---|---|
| | **PM** | **CMC, M** | **10g/L** | **1g/L** | **0,1g/L** | **0,01g/L** | **0,001g/L** |
| ***o*(C₁H₃)** | 612 | >1,5E-03 | 1,6E-02 | 1,6E-03 | 1,6E-04 | 1,6E-05 | 1,6E-06 |
| ***o*(C₃H₇)** | 640 | ∼1,5E-03 | 1,6E-02 | 1,6E-03 | 1,6E-04 | 1,6E-05 | 1,6E-06 |
| ***o*(C₇Hₗ₅)** | 696 | ∼1,5E-03 | 1,4E-02 | 1,4E-03 | 1,4E-04 | 1,4E-05 | 1,4E-06 |
| ***o*(C₁₂H₂₅)** | 766 | ∼1,5E-04 | 1,3E-02 | 1,3E-03 | 1,3E-04 | 1,3E-05 | 1,3E-06 |

### Exemple 12 : Effet de la solubilisation par la série p(COOH)₃-Ar4-o(CₙH₂₊₁) sur l'activité ATPase de BmrA

L'effet de la solubilisation par la série *p*(COOH)₃-Ar4-(CₙH₂ₙ₊₁) sur l'activité ATPasique de BmrA a été analysé dans les mêmes conditions que la figure 8.

La figure 9 montre l'effet des détergents utilisés (calixarènes comprenant une chaîne aliphatique méthyle, butyle, heptyle ou dodécyle) et des détergents de contrôle (FC12 and C12E8, un détergent non chargé) sur l'activité ATPasique avant, pendant et après solubilisation de BmrA. Comme classiquement observé, l'activité ATPasique décroit avec la solubilisation comme observée pour FC12 et C₁₂E₈.

Remarquablement, la comparaison entre les figures 8 et 9 montre que la molécule heptyle, à 1mg/m), solubilise plutôt efficacement BmrA alors que cette concentration, légèrement en dessous de la CMC, ne conduit pas à l'inactivation. Ceci est également vrai pour le composé dodécyle, à la même concentration, 1 mg/ml, 1/10 de CMC.

Les expériences de reconstitution de BmrA dans des bicouches lipidiques après élimination du détergent montrent une activité ATPasique totalement récupérée (losange noir dans la figure 9) indiquant que les molécules de l'invention préservent la topologie de BmrA dans un état fonctionnel.

### Exemple 13 : Extraction de la protéine BCRP d'origine humaine selon le procédé de l'invention.

La protéine BCRP, issu d'un gène humain, a été exprimée dans des cellules humaines HEK293 ou dans des cellules d'insectes *Sf*9 dans lesquelles le gêne correspondant a été transfecté. Cette protéine a également été solubilisée efficacement avec des dérivés de calixarènes de formule (I) comportant des chaînes heptyles ou plus longues, comme le montre la figure 10.

Ces expériences montrent que le procédé selon l'invention utilisant des calix[4]arènes trianioniques est un procédé efficace pour l'extraction d'une protéine membranaire donnée, en fonction du choix du calixarène de la série homologue qui, soit enrichi le surnageant, soit laisse la protéine dans le culot. Cette extraction peut éventuellement être suivie d'une étape de purification.

### Exemple 14: Effet de la solubilisation par les calixarènes selon l'invention sur l'activité enzymatique des transporteurs ABC.

Les protéines de la famille des transporteurs ABC sont très sensibles à la solubilisation, cette étape altérant souvent leur activité ATPase, du moins de façon réversible. Cette altération résulte d'un défaut de couplage entre les domaines NBDs (nucléotide-binding domain ou domaine de liaison-nucléotide) et TMDs (domaine transmembranaire).

Les effets de la solubilisation sur l'activité de la protéine BmrA ont été évalués avec divers calixarènes C4Cn, c'est-à-dire les calixarènes p(COOH)₃-Ar4-o(C,,H₂ₙ₊₁), mais aussi avec les détergents de contrôle tels que le Foscholine 12 (FC12) et le β-D-dodécyl maltopyranoside (DDM), puis les extraits obtenus (fractions de surnageant) ont été dialysés en présence de liposomes afin de restaurer un environnement membranaire, ce qui permet la récupération de l'activité de la protéine.

Les résultats représentés en figure 11 montrent que une fois solubilisée en présence de DDM ou de FC12, la protéine BmrA a perdu respectivement 95 et 99% de son activité ATPase (colonnes "DDM Sup. / DDM" et "FC12 Sup. / FC12" respectivement), par rapport à l'activité de BmrA que l'on peut mesurer dans les membranes natives ("BmrA Mb"). Néanmoins, cette activité ATPase est récupérée après élimination des détergents et reconstitution dans les liposomes (figure 11, colonnes "DDM Sup. / Lip." et "FC12 Sup. / Lip.").

Lorsque les dérivés calixarènes C4C3 et C4C7 sont utilisés successivement (voir l'exemple 15), environ 50% de l'activité ATPase est préservée, démontrant que ces détergents calixarènes permettent de maintenir la protéine membranaire en solution de façon plus rigide, contrairement aux détergents DDM et FC12. En effet, la structure rigide en cône des calixarènes de formule (I) permet la formation de micelles rigides qui constituent pour les protéines un milieu comparable à l'environnement membranaire, permettant une meilleure conservation de leur structure tridimensionnelle et donc de leur activité.

### Exemple 15 : Extraction et purification de protéines de la famille des transporteurs ABC avec des calixarènes de formule (I).

L'avantage de l'extraction différentielle de la protéine BmrA en utilisant successivement les calixarènes C4C3 puis C4C7, a permis d'enrichir la fraction de surnageant (comme montré en figure 12A), avant une purification ultérieure par chromatographie (figures 12B-E).

Le surnageant de C4C7 (échantillon "C4C7 S" de la figure 12A) a été soumis à une chromatographie de nickel-affinité (figures 12B et 12C), la protéine BmrA portant une agrafe hexahistidine à son extrémité N-terminale. La protéine s'associe ainsi à la résine et est éluée avec de l'imidazole, en présence de 0,3M de NaCl permettant de prévenir la chelation du Nickel par le calixarène trianionique.

Le même surnageant a également été soumis à une chromatographie de filtration sur gel en présence de Foscholine 12 (figure 12D), afin de vérifier l'état d'oligomérisation du transporteur. BmrA a été élué à un volume de 12 mL, correspondant à un poids moléculaire apparent de 200 000 Da, ce qui est une valeur compatible avec une forme dimérique du BmrA (2 x 65 000 Da) et une micelle de FC12 (70 000 Da). Au cours de cette étape, le calixarène C4C7 a été échangé avec FC12 et élué entre 15 et 25 mL, selon la détection par UV (OD à 280 nm en milliunités d'absorption mAU) et sur SDS-PAGE (encart dans la figure 12D). La fraction de BmrA (représentée par une étoile sur la figure 12D) se retrouve active (fraction a de la figure 12E), malgré la présence de FC12, ce qui suggère qu'une structure fonctionnelle de BmrA est préservée après solubilisation avec C4C7, et/ou des traces de C4C7 restent liées à la protéine. Une activité ATPase élevée a été récupérée après reconstitution de BmrA dans les liposomes (fraction b de la figure 12E). Ces résultats démontrent clairement qu'une protéine membranaire peut être purifiée en utilisant un couple de calixarènes de formule (I) et des étapes de chromatographie, préservant à chacune de ces étapes l'intégrité fonctionnelle de la protéine.

### Liste des références

[1]. Juliano, R. L., and Ling, V. (1976) A surface glycoprotein modulating drug permeability in Chinese hamster ovary cell mutants, Biochim Biophys Acta 455, 152-162.
[2]. Cole, S. P., Bhardwaj, G., Gerlach, J. H., Mackie, J. E., Grant, C. E., Almquist, K. C., Stewart, A. J., Kurz, E. U., Duncan, A. M., and Deeley, R. G. (1992) Overexpression of a transporter gene in a multidrug-resistant human lung cancer cell line, Science 258, 1650-1654.
[3]. Litman, T., Brangi, M., Hudson, E., Fetsch, P., Abati, A., Ross, D. D., Miyake, K., Resau, J. H., and Bates, S. E. (2000) The multidrug-resistant phenotype associated with overexpression of the new ABC half-transporter, MXR (ABCG2), J Cell Sci 113 ( Pt 11), 2011-2021.
[4]. Chami, M., Steinfels, E., Orelle, C., Jault, J. M., Di Pietro, A., Rigaud, J. L., and Marco, S. (2002) Three-dimensional structure by cryo-electron microscopy of YvcC, an homodimeric ATP-binding cassette transporter from Bacillus subtilis, J Mol Biol 315, 1075-1085.
[5]. Orelle, C., Dalmas, O., Gros, P., Di Pietro, A., and Jault, J. M. (2003) The conserved glutamate residue adjacent to the Walker-B motif is the catalytic base for ATP hydrolysis in the ATP-binding cassette transporter BmrA, J. Biol. Chem. 278, 47002-47008.
[6]. Lenoir, G., Menguy, T., Corre, F., Montigny, C., Pedersen, P. A., Thinès, D., le Maire, M., and Falson, P. (2002) Overproduction in yeast and rapid and efficient purification of the rabbit SERCA1a Ca2+-ATPase, Biochim. Biophys. Acta 1560, 67-83.
[7]. Steinfels, E., Orelle, C., Fantino, J. R., Dalmas, O., Rigaud, J. L., Denizot, F., Di Pietro, A., and Jault, J. M. (2004) Characterization of YvcC (BmrA), a multidrug ABC transporter constitutively expressed in Bacillus subtilis, Biochemistry 43, 7491-7502.
[8]. Smith, P. K., Krohn, R. I., Hermanson, G. T., Mallia, A. K., Gartner, F. H., Provenzano, M. D., Fujimoto, E. K., Goeke, N. M., Olson, B. J., and Klenk, D. C. (1985) Measurement of protein using bicinchoninic acid, Anal. Biochem. 150, 76-85.
[9]. Laemmli, U. K. (1970) Cleavage of structural proteins during the assembly of the head of bacteriophage T4, Nature 227, 680-685.
[10]. Decottignies, A., Grant, A. M., Nichols, J. W., de Wet, H., Mclntosh, D. B., and Goffeau, A. (1998) ATPase and multidrug transport activities of the overexpressed yeast ABC protein Yor1 p, J. Biol. Chem. 273, 12612-12622.

## Revendications

1. Procédé d'extraction sélective de protéines membranaires, c'est-à-dire de protéines associées aux membranes biologiques, comprenant une étape qui consiste à mettre en contact une solution aqueuse comprenant une ou plusieurs protéine(s) membranaire(s) de la protéine membranaire à extraire avec au moins un calixarène **caractérisé en ce que** la calixarène est un calixarène de formule (I) : 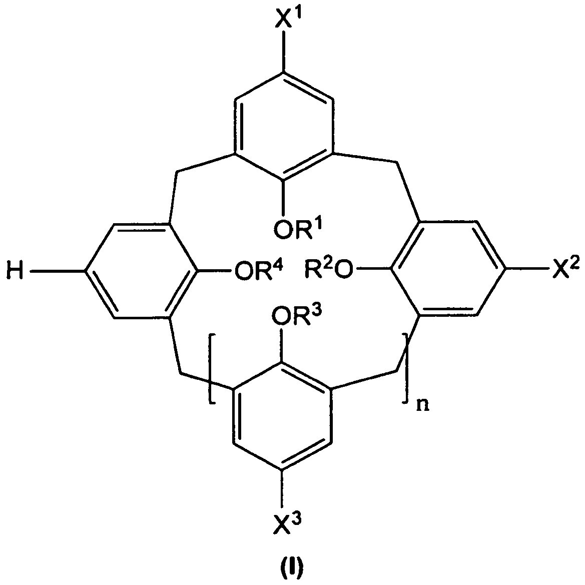 dans laquelle :
■ n est un nombre entier égal à 1, 3, 5 ou 6 ;
■ R¹, R², R³, R⁴ représentent, indépendamment l'un de l'autre, un atome d'hydrogène ; un groupe (C₁₋₁₂) alkyle linéaire ou ramifié éventuellement substitué par un ou plusieurs hétéroatomes choisis dans le groupe d'atomes O, S, N, P ; un groupe (C₁₋₁₂) alkyle linéaire ou ramifié éventuellement substitué par un groupe -COOR où R est un groupe (C₁₋₄) alkyle linéaire ou ramifié; un groupe aryle comprenant de 6 à 20 atomes de carbone ;
■ X¹, X², et X³ représentent, indépendamment l'un de l'autre, un groupe - (CH₂)ₘ-COOR' dans lequel
- m est un nombre entier allant de 0 à 10,
- R' représente un atome d'hydrogène ou un groupe (C₁₋₄) alkyle linéaire ou ramifié ;
ou l'un de ses sels pharmaceutiquement acceptables.

2. Procédé selon la revendication 1, dans lequel l'extraction sélective de la protéine membranaire est réalisée avec au moins un calixarène de formule (I) dans laquelle :
■ n est un nombre entier égal à 1 ;
■ R¹, R², R³, R⁴ représentent, indépendamment l'un de l'autre, un atome d'hydrogène ; un groupe (C₁₋₁₂) alkyle linéaire ou ramifié ;
■ X¹, X² et X³ représentent, indépendamment l'un de l'autre, un groupe - (CH₂)ₘ-COOR' dans lequel
- m est un nombre entier allant de 0 à 10,
- R' représente un atome d'hydrogène ;
ou l'un des sels pharmaceutiquement acceptables.

3. Procédé selon l'une des revendications 1 ou 2, dans lequel la protéine membranaire à extraire est présente dans une fraction membranaire biologique issue d'organisme procaryotique ou eucaryotique, sain ou altéré.

4. Procédé selon l'une quelconque des revendications 1 à 3, dans lequel la protéine membranaire est une protéine choisie dans le groupe comprenant les protéines de transport.

5. Procédé selon la revendication 4, dans lequel la protéine de transport est un transporteur ABC, choisi dans le groupe comprenant la glycoprotéines P (Pgp/ABCB1), MRP1/ABCC1, BCRP/ABCG2 et BmrA.

6. Procédé selon l'une quelconque des revendications 1 à 5, dans lequel l'étape de mise en contact d'une solution aqueuse comprenant la protéine membranaire à extraire avec au moins un calixarène de formule (I) est réalisée à un pH allant de 5.5 à 10.

7. Procédé selon l'une quelconque des revendications 1 à 6, dans lequel l'étape de mise en contact d'une solution aqueuse comprenant la protéine membranaire à extraire avec au moins un calixarène de formule (I) est réalisée à une température allant de 0 à 100°C.

8. Procédé selon l'une quelconque des revendications 1 à 7, dans lequel l'étape de mise en contact d'une solution aqueuse comprenant la protéine membranaire à extraire avec au moins un calixarène de formule (I) est réalisée à une concentration de calixarène allant de 10⁻⁶ à 10⁻² M.

9. Procédé selon l'une quelconque des revendications 1 à 8, dans lequel l'étape de mise en contact d'une solution aqueuse comprenant la protéine membranaire à extraire avec au moins un calixarène de formule (I) est réalisée avec des calixarènes en solution ou des calixarènes en agrégats colloïdaux.

10. Procédé selon la revendication 9, dans lequel l'agrégat colloïdal est choisi dans le groupe comprenant les micelles, les liposomes, les nanoparticules lipidiques.

11. Procédé selon l'une quelconque des revendications 1 à 10, dans lequel l'étape de mise en contact d'une solution aqueuse comprenant la protéine membranaire à extraire avec au moins un calixarène de formule (I) est réalisée éventuellement en présence d'au moins un co-soluté choisi dans le groupe comprenant,
i) les sels organiques et inorganiques choisi dans le groupe comprenant les sels pharmaceutiquement acceptables ;
ii) les petites molécules bioactives choisies dans le groupe des acides aminés, des vitamines, des lipides, des stéroïdes, des carbohydrates ou des métabolites ;
iii) les molécu!es bioactives oligomériques choisies dans le groupe des peptides, des oligonucléotides, des oligosaccharides ;
iv) les molécules biologiques polymériques choisies dans le groupe des protéines, polynucléotides et polysaccharides.

12. Procédé selon l'une quelconque des revendications 1 à 11, dans lequel l'étape de mise en contact est précédée par une étape dans laquelle :
- la protéine membranaire à extraire ou la fraction membranaire la contenant est solubilisée dans une solution tampon, et
- le calixarène de formule (I) est ajouté suivant les conditions décrites dans les revendications 6 à 11.

13. Procédé selon l'une quelconque des revendications 1 à 12, dans lequel les protéines complexées avec les calixarènes de formule (I) et les protéines non complexées sont séparées par centrifugation.

14. Protéine membranaire complexée avec au moins un calixarène de formule (I) tel que définie dans la revendication 1, ladite protéine membranaire complexée étant obtenue par un procédé d'extraction selon la revendication 13.

## Claims

1. A method for selectively extracting membrane proteins, that is to say proteins associated with biological membrane, comprising a stage which consists of contacting an aqueous solution comprising one or more membrane protein(s) of the membrane protein to be extracted with at least one calixarene, **characterized in that** the calixarene is a calixarene of formula (I): 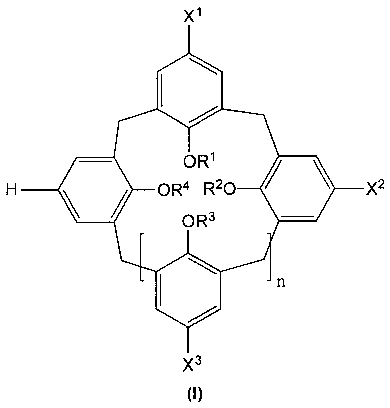 wherein:
■ n is an integer equal to 1, 3, 5 or 6;
■ R¹, R², R³ and R⁴ independently of one another represent a hydrogen atom, a linear or branched (C₁₋₁₂) alkyl group possibly substituted with one or more hetero atoms selected from the group of O, S, N and P atoms, a linear or branched (C₁₋₁₂) alkyl group possibly substituted with a -COOR group where R is a linear or branched (C₁₋₄) alkyl group, or an aryl group comprising from 6 to 20 carbon atoms; and
■ X¹, X² , and X³ independently of one another represent a group -(CH₂)ₘ-COOR' in which
- m is a integer ranging from 0 to 10, and
- R' represents a hydrogen atom or a linear or branched (C₁₋₄) alkyl group;
or one of the pharmaceutically acceptable salts thereof.

2. The method according to claim 1, wherein the selective extraction of the membrane protein is effected using at least one calixarene of formula (I) in which:
■ n is an integer equal to 1;
■ R¹, R², R³, R⁴ independently of one another represent a hydrogen atom or a linear or branched (C₁₋₁₂) alkyl group; and
■ X¹, X² and X³ independently of one another represent a -(CH₂)ₘ-COOR' group in which
- m is a integer ranging from 0 to 10, and
- R' represents a hydrogen atom;
or one of the pharmaceutically acceptable salts.

3. The method according to claim 1 or 2, wherein the membrane protein to be extracted is present in a biological membrane fraction derived from a prokaryotic or eukaryotic, healthy or impaired organism.

4. The method according to any one of claims 1 to 3, wherein the membrane protein is a protein selected from the group comprising the transport proteins.

5. The method according to claim 4, wherein the transport protein is an ABC transporter selected from the group comprising the P glycoproteins (Pgp/ABCB1), MRP1/ABCC1, BCRP/ABCG2 and BmrA.

6. The method according to any one of claims 1 to 5, wherein the stage of contacting an aqueous solution comprising the membrane protein to be extracted with at least one calixarene of formula (I) is effected at a pH ranging from 5.5 to 10.

7. The method according to any one of claims 1 to 6, wherein the stage of contacting an aqueous solution comprising the membrane protein to be extracted with at least one calixarene of formula (I) is effected at a temperature ranging from 0 to 100°C.

8. The method according to any one of claims 1 to 7, wherein the stage of contacting an aqueous solution comprising the membrane protein to be extracted with at least one calixarene of formula (I) is effected at a calixarene concentration ranging from 10⁻⁶ to 10⁻² M.

9. The method according to any one of claims 1 to 8, wherein the stage of contacting an aqueous solution comprising the membrane protein to be extracted with at least one calixarene of formula (I) is effected with calixarenes in solution or calixarenes in colloidal aggregates.

10. The method according to claim 9, wherein the colloidal aggregate is selected from the group comprising micelles, liposomes, lipid nanoparticles.

11. The method according to any one of claims 1 to 10, wherein the stage of contacting an aqueous solution comprising the membrane protein to be extracted with at least one calixarene of formula (I) is possibly effected in the presence of at least one co-solute selected from the group comprising:
i) organic and inorganic salts selected from the group comprising the pharmaceutically acceptable salts;
ii) small biologically active molecules selected from the group of the amino acids, vitamins, lipids, steroids, carbohydrates or metabolites;
iii) oligomeric biologically active molecules selected from the group of the peptides, oligonucleotides and oligosaccharides;
iv) polymeric biological molecules selected from the group of the proteins, polynucleotides and polysaccharides.

12. The method according to any one of claims 1 to 11, wherein the contacting stage is preceded by a stage in which:
- the membrane protein to be extracted or the membrane fraction containing it is solubilized in a buffer solution, and
- the calixarene of formula (I) is added according to the conditions described in claims 6 to 11.

13. The method according to any one of claims 1 to 12, wherein the proteins complexed with the calixarenes of formula (I) and the non-complexed proteins are separated by centrifugation.

14. A membrane protein complexed with the calixarenes of formula (I) as defined in claim 1, said complexed membrane protein being obtained by a extracting method according to claim 13.

## Patentansprüche

1. Verfahren zur selektiven Extraktion von Membranproteinen, d.h. von Proteinen, die mit biologischen Membranen assozüert sind, welches einen Schritt umfasst, der darin besteht, eine wässrige Lösung, die ein oder mehrere Membranproteine des zu extrahierenden Membranproteins enthält, mit mindestens einem Calixaren in Kontakt zu bringen, **dadurch gekennzeichnet, dass** das Calixaren ein Calixaren der Formel (I) ist: 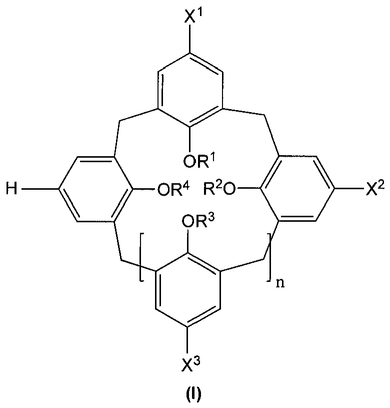 wobei:
■ n eine ganze Zahl gleich 1, 3, 5 oder 6 ist;
■ R¹, R², R³ und R⁴ unabhängig voneinander darstellen: ein Wasserstoffatom; eine lineare oder verzweigte (C₁₋₁₂)-Alkylgruppe, die gegebenfalls duch ein oder mehrere Heteroatome substituiert wird, die aus der Gruppe der O, S, N, P -Atome gewählt werden; eine lineare oder verzweigte (C₁₋₁₂)-Alkylgruppe, die gegebenenfalls durch eine -COOR Gruppe substituiert wird, wobei R eine lineare oder verzweigte (C₁₋₄)-Alkylgruppe ist; eine Arylgruppe, die 6 bis 20 Kohlenstoffatome umfasst; und
■ X¹, X², und X³ unabhängig voneinander eine -(CH_{z})ₘ COOR'-Gruppe darstellen, in der
- m eine ganze Zahl von 0 bis 10 ist und
- R' ein Wasserstoffatom oder eine lineare oder verzweigte (C₁₋₄)-Alkylgruppe darstellt;
oder eines der pharmazeutisch akzeptablen Salze hiervon.

2. Verfahren nach Anspruch 1, wobei die selektive Extraktion des Membranproteins unter Verwendung von mindestens eines Calixaren der Formel (I) durchgeführt wird, wobei :
■ n eine ganze Zahl gleich 1 ist;
**■** R¹, R², R³, R⁴ unabhängig voneinander ein Wasserstoffatom; eine lineare oder verzweigte (C₁₋₁₂)-Alkylgruppe darstellen;
■ X¹, X² und X³ unabhängig voneinander eine -(CH₂)ₘ-COOR'-Gruppe darstellen, in der
- m eine ganze Zahl von 0 bis 10 ist,
- R' ein Wasserstoffatom darstellt;
oder eines der pharmazeutisch akzeptablen Salze hiervon.

3. Verfahren nach Anspruch 1 oder 2, wobei das zu extrahierende Membranprotein in einer aus einem gesunden oder veränderten prokaryotischen oder eukaryotischen Organismus stammenden biologischen Membranfraktion vorliegt.

4. Verfahren nach einem der Ansprüche 1 bis 3, wobei das Membranprotein ein Protein ist, das aus der Gruppe gewählt wird, welche die Transportproteine umfasst.

5. Verfahren nach Anspruch 4, wobei das Transportprotein ein ABC-Transporter ist, der aus der Gruppe gewählt wird, die P-Glykoproteine (Pgp/ABCB1), MRP1/ABCC1, BCRP/ABCG2 und BmrA umfasst.

6. Verfahren nach einem der Ansprüche 1 bis 5, wobei der Schritt, in dem die das zu extrahierende Membranprotein enthaltende wässrige Lösung mit mindestens einem Calixaren der Formel (1) in Kontakt gebracht wird, bei einem pH-Wert von 5.5 bis 10 durchgeführt wird.

7. Verfahren nach einem der Ansprüche 1 bis 6, wobei der Schritt, in dem die das zu extrahierende Membranprotein enthaltende wässrige Lösung mit mindestens einem Calixaren der Formel (1) in Kontakt gebracht wird, bei einer Temperatur von 0 bis 100 °C durchgeführt wird.

8. Verfahren nach einem der Ansprüche 1 bis 7, wobei der Schritt, in dem die das zu extrahierende Membranprotein enthaltende wässrige Lösung mit mindestens einem Calixaren der Formel (1) in Kontakt gebracht wird, bei einer Calixaren-Konzentration von 10⁻⁶ bis 10⁻² M durchgeführt wird.

9. Verfahren nach einem der Ansprüche 1 bis 8, wobei der Schritt, in dem die das zu extrahierende Membranprotein enthaltende wässrige Lösung mit mindestens einem Calixaren der Formel (1) in Kontakt gebracht wird, mit Calixarenen in Lösung oder Calixarenen in kolloidalen Aggregaten durchgeführt wird.

10. Verfahren nach Anspruch 9, wobei das kolloidale Aggregat aus der Gruppe gewählt wird, die Mizellen, Liposome, Lipid-Nanopartikel umfasst.

11. Verfahren nach einem der Ansprüche 1 bis 10, wobei der Schritt, in dem die das zu extrahierende Membranprotein enthaltende wässrige Lösung mit mindestens einem Calixaren der Formel (I) in Kontakt gebracht wird, gegebenenfalls bei Vorhandensein von mindestens einem zweiten Gelösten aus der Gruppe gewählt, die folgende umfasst:
i) organische und anorganische Salze aus der Gruppe gewählt, die pharmazeutisch akzeptable Salze umfasst;
ii) kleine biologisch aktive Moleküle aus der Gruppe gewählt, die Aminosäuren, Vitamine, Lipide, Steroide, Kohlenhydrate oder Metaboliten umfasst;
iii) oligomere biologisch aktive Moleküle aus der Gruppe der Peptide, Oligonukleotide und Oligosaccharide gewählt;
iv) polymere biologische Moleküle aus der Gruppe der Proteine, Polynukleotide und Polysaccharide gewählt.

12. Verfahren nach einem der Ansprüche 1 bis 11, wobei der Schritt des Inkontaktbringens auf einen Schritt folgt, in dem:
- das zu extrahierende Membranprotein oder die es enthaltende Membranfraktion in einer Pufferlösung gelöst wird, und
- das Calixaren nach Formel (I) unter den in den Ansprüchen 6 bis 11 beschriebenen Bedingungen zugefügt wird.

13. Verfahren nach einem der Ansprüche 1 bis 12, wobei die mit dem Calixaren nach Formel (I) komplexierten Proteine und die nicht komplexierten Proteine durch Zentrifugieren getrennt werden.

14. Ein mit mindestens einem Calixaren nach Formel (I) gemäss der Definition in Anspruch 1 komplexiertes Membranprotein, wobei besagtes komplexiertes Membranprotein durch das Extraktionsverfahren nach Anspruch 13 erhalten wird.
